# EUROPEAN PATENT SPECIFICATION

(11) **EP 2 721 163 B1**
(45) Date of publication and mention of the grant of the patent: **21.12.2016**
(21) Application number: 12732891.2
(22) Date of filing: 15.06.2012
(51) Int. Cl.: C12P 7/06, C12P 7/16, C12P 7/36

(54) **CO-PRODUCTS FROM BIOFUEL PRODUCTION PROCESSES AND METHODS OF MAKING**
NEBENPRODUKTE AUS BIOKRAFTSTOFFPRODUKTIONSVERFAHREN UND HERSTELLUNGSVERFAHREN
COPRODUITS PROVENANT DE PROCÉDÉS DE PRODUCTION DE BIOCARBURANTS ET LEURS PROCÉDÉS DE FABRICATION

(30) Priority: 17.06.2011 US 201161498389 P
(43) Date of publication of application: 23.04.2014
(73) Proprietor: Butamax Advanced Biofuels LLC, Wilmington, Delaware 19880-0356 (US)
(72) Inventor: LOWE, David, J., Wilmington, DE 19808 (US); ROESCH, Brian, Michael, Middletown, DE 19709 (US)
(74) Representative: Towler, Philip Dean
(86) International application number: PCT/US2012/000290
(87) International publication number: WO 2012/173660

(56) References cited:
- WO-A1-2009/114451
- WO-A2-02/074895
- WO-A2-2005/021742
- WO-A2-2005/087937
- WO-A2-2007/121100
- WO-A2-2008/061120
- WO-A2-2011/160030
- US-A1- 2007 243 235
- US-A1- 2007 243 592
- US-A1- 2009 259 018

## Description

### BACKGROUND OF THE INVENTION

### Field of the Invention

This invention relates to methods of generating co-products and compositions useful as co-products derived from biofuel production processes. For example, the invention relates to co-products for animal feed from at least one process feedstream, such as fatty acids from oil hydrolysis, lipids from evaporation of thin stillage, syrup, distillers grains, distillers grains and solubles, solids from a mash before fermentation, and solids from a whole stillage after fermentation.

### Background Art

Biofuels are a wide range of fuels which in some way are derived from biomass. Biofuels are gaining increased public and scientific attention, driven by factors such as oil price spikes, the need for increased energy security, concern over greenhouse gas emissions from fossil fuels, and government subsidies. Biofuels provided 1.8% of the world's transport fuel in 2008. Investment in biofuels production capacity exceeded $4 billion worldwide in 2007 and is growing. According to the International Energy Agency, biofuels have the potential to meet more than a quarter of world demand for transportation fuels by 2050.

In addition to biofuels, biofuel production processes (e.g., ethanol and butanol production processes) also produce by-products such as dried distillers grains and solubles (DDGS) ("distillers co-products"). Distillers co-products can provide an economic benefit to the alcohol producer. For example, DDGS may be converted for use as animal feed, food ingredients, and industrial products. Development of alternative uses for distillers co-products minimizes the need and costs for disposal of fermentation by-products. US2007/0243235 discloses a method of generating a distillers co-products of a butanol production process, such as distillers wet (DWS) or dried grains (DDG) which may be combined with distillers soluble to form e.g. distillers dried grains with solubles (DDGS).

There may be variability in the nutrient profile of distillers co-products. This variability may depend on, for example, different feedstock sources used in the biofuel production process. Therefore, there is a need to develop methods for generating distillers co-products and methods for nutritionally customizing the distillers co-products for a particular animal feed or animal feed market (e.g., livestock, ruminant, cattle, dairy animal, swine, goat, sheep, poultry, equine, aquaculture, or domestic pet such as dogs, cats, and rabbits). For example, lysine is an important nutritional additive to animal feed because it is a limiting amino acid when optimizing the growth of certain animals such as pigs and chickens for the production of meat. Lysine supplementation allows for the use of lower-cost plant protein (maize, for instance, rather than soy) while maintaining high growth rates and limiting the pollution from nitrogen excretion. Further, consistent reproducible quality distillers co-products with an extended shelf life are needed.

The present invention satisfies these and other needs, and provides further related advantages, as will be made apparent by the description of the embodiments that follow.

### SUMMARY OF THE INVENTION

The present invention is defined in the appended claims. Disclosed is a method of generating a distillers co-products comprising providing an alcohol production process with at least two process feedstreams, wherein the at least two process feedstreams are combined to generate a distillers co-products. In some embodiments, the at least two process feedstreams include at least two of (i) fatty acids from oil hydrolysis, (ii) lipids from evaporation of thin stillage, (iii) syrup, (iv) distillers grains (DG), (v) distillers grains and solubles (DGS), (vi) solids from a mash before fermentation; (vii) solids from a whole stillage after fermentation, (viii) oil, and (ix) microorganism. In some embodiments, the fatty acids are from corn oil hydrolysis. In some embodiments, the DG are dried distillers grains (DDG) or wet distillers grains (WDG). In some embodiments, the DGS are dried distillers grains and solubles (DDGS) or wet distillers grains and solubles (WDGS). In some embodiments, the alcohol production process is a butanol production process.

Disclosed are methods of generating a distillers co-products for animal feed from an alcohol process comprising providing an alcohol (e.g., butanol) production process with at least one process feedstream, wherein the at least one process feedstream is used to generate a distillers co-products for animal feed with an improved crude protein and crude fat content. In some embodiments, there may be at least two or at least three process feedstreams, wherein the process feedstreams are combined to generate a distillers co-products for animal feed. In some embodiments, the process feedstreams may include (i) fatty acids, (ii) lipids, (iii) syrup, (iv) distillers grains (DG), (v) distillers grains and solubles (DGS), (vi) solids from a mash before fermentation; (vii) solids from a whole stillage after fermentation; (viii) oil, and/or (ix) microorganism. In some embodiments, the fatty acids may be derived from oil hydrolysis. In some embodiments, the fatty acids are from corn oil hydrolysis (COFA). In some embodiments, the lipids may be derived from evaporation of thin stillage. In some embodiments, the DG are dried distillers grains (DDG), wet distillers grains (WDG), dried distillers grains and solubles (DDGS), or wet distillers grains and solubles (WDGS). In some embodiments, the animal feed may have a crude protein content of at least about 20%. In some embodiments, the animal feed may have a crude protein content of at least about 25%. In some embodiments, the distillers co-products for animal feed has a crude fat content of less than 15%. In some embodiments, the distillers co-products for animal feed has a crude fat content of less than 10%. In some embodiments, the distillers co-products for animal feed have a fatty acid content of less than about 10%. In some embodiments, the fatty acid content provides additional energy and nutrient for an animal feed composition that comprises the distillers co-products for animal feed. In some embodiments, the alcohol production process is a butanol production process. In some embodiments, butanol produced in the butanol production process is used as a solvent wash for at least one feedstream of the butanol production process. In some embodiments, a first feedstream is combined with a second feedstream to produce a distillers co-products for animal feed with increased storage stability. In some embodiments, the distillers co-products for animal feed has an improved color profile.

Disclosed is a distillers co-products for animal feed composition comprising at least about 20% crude protein by weight of the distillers co-products and less than about 10% crude fat by weight, wherein the composition has an improved nutrient profile for an animal feed or animal feed market. In some embodiments, the distillers co-products for animal feed composition further comprising less than about 10% fatty acids by weight. In some embodiments, the distillers co-products for animal feed composition further comprising less than about 10% fatty acid ester by weight. In some embodiments, the distillers co-products for animal feed composition further comprising no more than about 5% lysine by weight. In some embodiments, the distillers co-products for animal feed composition has a nutrient profile for cattle, dairy, livestock, swine, poultry, equine, aquaculture, or domestic pet feed market. In some embodiments, the distillers co-products for animal feed composition further comprising supplementing the distillers co-products for animal feed composition with one or more additional components selected from amino acids, vitamins, minerals, nutrient, flavor enhancer, digestion stimulant, or color enhancer.

Disclosed are methods of generating a distillers co-products for animal feed, comprising providing a butanol production process with at least three process feedstreams, wherein the at least three process feedstreams are combined to generate a distillers co-products for animal feed having a crude protein content of at least about 20%. In some embodiments, the process feedstreams include at least three of (i) fatty acids from oil hydrolysis, (ii) lipids from evaporation of thin stillage, (iii) syrup, (iv) distillers grains (DG), (v) distillers grains and solubles (DGS), (vi) solids from a mash before fermentation; and (vii) solids from a whole stillage after fermentation. In some embodiments, the fatty acids are from corn oil hydrolysis (COFA). In some embodiments, the DG are dried distillers grains (DDG), wet distillers grains (WDG), dried distillers grains and solubles (DDGS) or wet distillers grains and solubles (WDGS). In some embodiments, the distillers co-products for animal feed has a crude fat content of less than 10%. In some embodiments, the distillers co-products for animal feed have a fatty acid content of less than about 10%.

Disclosed are methods of producing a high value animal feed component from a butanol production process, comprising providing a butanol production process with at least one process feedstream to optimize crude protein and crude fat content for an animal feed or animal feed market.

Disclosed are methods of mitigating the impact of fermentation contaminants on the production of a distillers co-products for animal feed, comprising separating at least one feedstream of a butanol production process prior to fermentation.

Disclosed are methods of reducing mycotoxin contamination in a distillers co-products for animal feed comprising separating feedstreams of a butanol production process contributing to distillers co-products for animal feed production, and eliminating or purifying feedstreams with mycotoxin contamination potential.

Disclosed are methods of reducing lipid content variability of a distillers co-products for animal feed comprising separating feedstreams of a butanol production process contributing to a distillers co-products for animal feed production, and combining the feedstreams to achieve a controlled lipid content.

Disclosed are methods of increasing triglyceride content of a distillers co-products for animal feed comprising combining higher triglyceride-containing feedstreams of a butanol production process at an increasing ratio as compared to lower triglyceride-containing streams for a particular distillers co-products for animal feed composition.

Disclosed is a distillers co-products for animal feed produced by a method of the invention.

Disclosed are distillers co-products for animal feed composition comprising at least about 20% or at least about 25% crude protein and less than about 10% or less than about 15% crude fat, wherein the composition has a nutrient profile for an animal feed or animal feed market. In some embodiments, the distillers co-products for animal feed composition further comprises less than about 10% fatty acid isobutyl ester. In some embodiments, the distillers co-products for animal feed composition further comprises less than about 5% lysine. In some embodiments, the composition has a nutrient profile for a swine feed market, a dairy feed market (e.g., dairy cow feed market), a cattle feed market, a poultry feed market (e.g., chicken feed market), an equine feed market, an aquaculture feed market, a livestock feed market, and/or a domestic pet feed market

An end product of the instant fermentation processes is an product alcohol, for example, butanol or ethanol. The end product produced according to the processes can be separated and/or purified from the fermentation media. Methods for separation and purification are known, for example by subjecting the media to extraction, pervaporation, or distillation. In some embodiments, a mash can be separated by for example centrifugation into the liquid phase and solids phase and end products such as alcohol and solids recovered. The alcohol can be recovered by means such as distillation and molecular sieve dehydration or ultra-filtration.

In some embodiments, the yield of butanol can be greater than about 8%, about 10%, about 12%, about 14%, about 16% or about 18% by volume.

In some embodiments, the butanol is 1-butanol (1-BuOH), 2-butanol (2-BuOH), tertiary-butanol (tert-BuOH), and/or isobutanol (iBuOH, i-BuOH, or I-BUOH), either individually or as mixtures thereof.

Further features and advantages of embodiments described herein, as well as the structure and operation of various embodiments, are described in detail below with reference to the accompanying drawings. It is noted that the embodiments described below are not limited to the specific embodiments described herein. Such embodiments are presented herein for illustrative purposes only. Additional embodiments will be apparent to persons skilled in the relevant art based on the teachings contained herein.

### DESCRIPTION OF THE DRAWINGS

Figure 1a schematically illustrates process feedstreams of an exemplary method and system of the present invention.
Figure 1b illustrates an example of a wet milling process for processing feedstock.
Figure 1c illustrates an example of a dry milling process for processing feedstock.
Figure 2 schematically illustrates an exemplary method and system of the present invention in which solids are removed before fermentation.
Figure 3 schematically illustrates an exemplary alternative method and system of the present invention in which solids are removed before fermentation.
Figure 4 schematically illustrates an exemplary alternative method and system of the present invention, in which solids and oil are removed before fermentation.
Figure 5 schematically illustrates an exemplary alternative method and system of the present invention in which solids are removed before fermentation.
Figure 6 schematically illustrates an exemplary alternative method and system of the present invention for the production of a product alcohol.
Figure 7 schematically illustrates an exemplary alternative method and system of the present invention for the production of a product alcohol in which solids are removed before fermentation.
Figure 8 schematically illustrates another exemplary alternative method and system of the present invention for the production of a product alcohol in which solids are removed before fermentation.
Figure 9 illustrates the mass balance of process feedstream of exemplary distillers co-products for animal feed.

In the drawings, like reference numbers indicate identical or functionally similar elements.

### DETAILED DESCRIPTION OF THE INVENTION

Unless defined otherwise, all technical and scientific terms used herein have the same meaning as commonly understood by one of ordinary skill in the art to which this invention belongs. In case of conflict, the present application including the definitions will control. Also, unless otherwise required by context, singular terms shall include pluralities and plural terms shall include the singular. Unless indicated otherwise, the percentage values described herein are in terms of the percentage of weight of a process feedstream, distillers co-products, or compositions, as appropriate.

In order to further define this invention, the following terms and definitions are herein provided.

As used herein, the terms "comprises," "comprising," "includes," "including," "has," "having," "contains," or "containing," or any other variation thereof, will be understood to imply the inclusion of a stated integer or group of integers but not the exclusion of any other integer or group of integers. For example, a composition, a mixture, a process, a method, an article, or an apparatus that comprises a list of elements is not necessarily limited to only those elements but can include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances, that is, occurrences of the element or component. Therefore, "a" or "an" should be read to include one or at least one, and the singular word form of the element or component also includes the plural unless the number is obviously meant to be singular.

The term "invention" or "present invention" as used herein is a non-limiting term and is not intended to refer to any single embodiment of the particular invention but encompasses all possible embodiments as presently claimed.

As used herein, the term "about" modifying the quantity of an ingredient or reactant of the invention employed refers to variation in the numerical quantity that can occur, for example, through typical measuring and liquid handling procedures used for making concentrates or solutions in the real world; through inadvertent error in these procedures; through differences in the manufacture, source, or purity of the ingredients employed to make the compositions or to carry out the methods; and the like. The term "about" also encompasses amounts that differ due to different equilibrium conditions for a composition resulting from a particular initial mixture. Whether or not modified by the term "about," the claims include equivalents to the quantities. In some embodiments, the term "about" means within 10% of the reported numerical value, alternatively within 5% of the reported numerical value.

"Alcohol" or "product alcohol" as used herein refers to any alcohol that can be produced by a microorganism in a fermentation process that utilizes biomass as a source of fermentable carbon substrate. Alcohols include, but are not limited to, C₁ to C₈ alkyl alcohols. In some embodiments, the alcohols are C₂ to C₈ alkyl alcohols. In other embodiments, the alcohols are C₂ to C₅ alkyl alcohols. It will be appreciated that C₁ to C₈ alkyl alcohols include, but are not limited to, methanol, ethanol, propanol, butanol, and pentanol. Likewise C₂ to C₈ alkyl alcohols include, but are not limited to, ethanol, propanol, butanol, and pentanol.

"Butanol" as used herein refers with specificity to the butanol isomers: 1-butanol (1-BuOH), 2-butanol (2-BuOH), tertiary-butanol (tert-BuOH), and/or isobutanol (iBuOH, i-BuOH, or I-BUOH), either individually or as mixtures thereof.

"Biomass" as used herein refers to a natural product containing hydrolyzable polysaccharides that provide fermentable sugars including any sugars and starch derived from natural resources such as corn, sugar cane, wheat, cellulosic or lignocellulosic material and materials comprising cellulose, hemicellulose, lignin, starch, oligosaccharides, disaccharides and/or monosaccharides, and mixtures thereof. Biomass can also comprise additional components such as protein and/or lipids. Biomass can be derived from a single source or biomass can comprise a mixture derived from more than one source. For example, biomass can comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, wood and forestry waste. Examples of biomass include, but are not limited to, corn grain, corn cobs, corn fiber, crop residues such as corn husks, corn stover, grasses, wheat, rye, wheat straw, barley, barley straw, hay, rice straw, switchgrass, waste paper, sugar cane bagasse, sorghum, sugar cane, soy, components obtained from milling of grains, trees, branches, roots, leaves, wood chips, sawdust, shrubs and bushes, vegetables, fruits, flowers, animal manure, and mixtures thereof. For example, mash, juice, molasses, or hydrolysate can be formed from biomass by any processing known in the art for processing the biomass for purposes of fermentation such as by milling, treating, and/or liquefying and comprises fermentable sugar and can comprise water. For example, cellulosic and/or lignocellulosic biomass can be processed to obtain a hydrolysate containing fermentable sugars by any method known to one skilled in the art. A low ammonia pretreatment is disclosed in U.S. Patent Application Publication No. 2007/0031918A1. Enzymatic saccharification of cellulosic and/or lignocellulosic biomass typically makes use of an enzyme consortium for breaking down cellulose and hemicellulose to produce a hydrolysate containing sugars including glucose, xylose, and arabinose. Saccharification enzymes suitable for cellulosic and/or lignocellulosic biomass are reviewed in Lynd, et al. (Microbiol. Mol. Biol. Rev. 66:506-577, 2002).

"Feedstock" as used herein means a feed in a fermentation process, the feed containing a fermentable carbon source with or without undissolved solids, and where applicable, the feed containing the fermentable carbon source before or after the fermentable carbon source has been liberated from starch or obtained from the breakdown of complex sugars by further processing such as by liquefaction, saccharification, or other process. Feedstock includes or is derived from a biomass. Suitable feedstocks include, but are not limited to, rye, wheat, corn, corn mash, cane, cane mash, barley, cellulosic material, lignocellulosic material, or mixtures thereof.

"Fermentable carbon source" or "fermentable carbon substrate" as used herein means a carbon source capable of being metabolized by microorganisms for the production of fermentative alcohol. Suitable fermentable carbon sources include, but are not limited to, monosaccharides such as glucose or fructose; disaccharides such as lactose or sucrose; oligosaccharides; polysaccharides such as starch or cellulose; C5 sugars such as xylose and arabinose; one carbon substrates including methane; and mixtures thereof.

As used herein, the term "fermentable sugars" refers to one or more sugars (e.g., oligosaccharides and monosaccharides) that can be converted into end products by fermentation with a fermenting microorganism.

"Sugar" as used herein refers to oligosaccharides, disaccharides, monosaccharides, and/or mixtures thereof. The term "saccharide" also includes carbohydrates including starches, dextrans, glycogens, cellulose, pentosans, as well as sugars.

As used herein, the term "milled" refers to plant material that has been reduced in size, such as by grinding, crushing, fractionating or by any other means of particle size reduction.

As used herein, the term "mash" refers to a mixture of a fermentable substrate in liquid used in the production of a fermented product. This term refers to any stage of the fermentation from the initial mixing of the fermentable substrate with one or more starch hydrolyzing enzymes and fermenting organisms through the completion of the fermentation run. From time to time as used herein, the term "mash" and "feedstock slurry" can be used synonymously.

As used herein, the term "fermentation" refers to the enzymatic and/or anaerobic breakdown of organic substances by microorganisms to produce simpler organic compounds. While fermentation may occur under anaerobic conditions, it is not intended that the term be solely limited to strict anaerobic conditions, as fermentation may also occur under aerobic (e.g., in the presence of oxygen) or microaerobic conditions.

"Fermentation broth" as used herein means the mixture of water, sugars (fermentable carbon sources), dissolved solids, optionally microorganisms producing alcohol, product alcohol, and all other constituents of the material held in the fermentation vessel or fermentor in which product alcohol is being made by the reaction of sugars to alcohol, water, and carbon dioxide (CO₂) by the microorganisms present. As used herein the term "fermentation broth" can be used synonymously with "fermentation medium."

As used herein, the term "distillers co-products" refers to by-products from an alcohol (e.g., butanol or ethanol) production process that can be isolated before or during fermentation. Distillers co-products include non-fermentable products remaining after alcohol is removed from a fermented mash and solids isolated from a mash. As used herein, distillers co-products may be used in a variety of animal feed and non-animal feed applications. Examples of distillers co-products include, but are not limited to, fatty acids from oil hydrolysis, lipids from evaporation of thin stillage, syrup, distillers grains, distillers grains and solubles, solids from a mash before fermentation, and solids from a whole stillage after fermentation, biodiesel, and acyl glycerides.

As used herein, the term "distillers co-products for animal feed" refers to distillers co-products that are suitable for use in or as animal feed. Examples of distillers co-products for animal feed include, but are not limited to, fatty acids from oil hydrolysis, lipids from evaporation of thin stillage, syrup, distillers grains, distillers grains and solubles, solids from a mash before fermentation, and solids from a whole stillage after fermentation.

As used herein, the terms "distillers grains" or "DG" refer to the non-fermentable products remaining after alcohol (e.g., ethanol and/or butanol) is removed from a fermented mash. Distillers grains that are dried are known as "distillers dried grains" or "DDG." Distillers grains that are not dried are known as "wet distillers grains" or "WDG."

As used herein, the terms "distillers grains and solubles" or "DGS" refer to the non-fermentable products remaining after alcohol (e.g., ethanol and/or butanol) is removed from a fermented mash that have been blended with solubles. Distillers grains and solubles that are dried are known as "distillers dried grains and solubles" or "DDGS." Distillers grains and solubles that are not dried are known as "wet distillers grains and solubles" or "WDGS."

As used herein, the term "fatty acids from hydrolyzing oil" used in regard to a process feedstream means a fatty acid by-product produced by hydrolyzing an oil during an alcohol (e.g., ethanol or butanol) production process. The fatty acid by-product can be formed by centrifugation and hydrolysis of whole stillage following fermentation in an alcohol (e.g., ethanol or butanol) production process. The fatty acid by-product can be, for example, produced by hydrolyzing corn oil to form "fatty acids from hydrolyzing corn oil."

As used herein, the term "lipid" refers to any of a heterogeneous group of fats and fatlike substances including fatty acids, neutral fats, waxes, and steroids, which are water-insoluble and soluble in nonpolar solvents. Examples of lipids include monoglycerides, diglycerides, triglycerides, and phospholipids.

As used herein, the term "lipids from evaporation" used in reference to a process feedstream means a lipid by-product produced by evaporation and centrifugation of thin stillage following fermentation in an alcohol (e.g., ethanol or butanol) production process.

As used herein, the term "syrup" or "condensed distillers solubles" (CDS) used in reference to a process feedstream means a by-product produced by evaporation of thin stillage following fermentation in an alcohol (e.g., ethanol or butanol) production process.

As used herein, "process feedstream" refers to any by-product formed before or during an alcohol (e.g., ethanol or butanol) production process. Examples of process feedstreams include, but are not limited to, COFA, lipids from evaporation, syrup, DG, DDG, WDG, DGS, DDGS, and WDGS. Another example of a process feedstream are the solids removed (e.g., by centrifugation) from a mash before fermentation in an ethanol or butanol production process (e.g., the solids removed from a corn mash before fermentation). These solids are referred to herein as "wet cake" when they have not been dried, and are referred to herein as "dry cake" when they have been dried. Another example of a process feedstream are the solids removed (e.g., by centrifugation) from whole stillage following fermentation in an alcohol (e.g., ethanol or butanol) production process. These solids are referred to herein as "WS wet cake" when they have not been dried, and are referred to herein as "WS dry cake" when they have been dried.

The term "aqueous phase" as used herein refers to the aqueous phase of a biphasic mixture obtained by contacting a fermentation broth with an extractant. In some embodiments, the term "fermentation broth" may refer to the aqueous phase in biphasic fermentative extraction. In addition, undissolved solids (e.g., grain solids) can be present in the fermentation broth, such that the biphasic mixture includes the undissolved solids which may be dispersed in the aqueous phase.

The term "organic phase" as used herein refers to the non-aqueous phase of a biphasic mixture obtained by contacting a fermentation broth with an extractant.

"Extractant" as used herein means a solvent used to extract an alcohol such as butanol or used to extract an alcohol ester (e.g., produced by catalysis of an alcohol and a carboxylic acid or lipid). From time to time, as used herein the term "solvent" may be used synonymously with "extractant." In some embodiments, extractants may be organic solvents. In some embodiments, extractants may be water-immiscible organic solvents.

The terms "water-immiscible" refer to a chemical component such as an extractant or solvent, which is incapable of mixing with an aqueous solution such as a fermentation broth, in such a manner as to form one liquid phase.

The term "carboxylic acid" as used herein refers to any organic compound with the general chemical formula -COOH in which a carbon atom is bonded to an oxygen atom by a double bond to make a carbonyl group (-C=O) and to a hydroxyl group (-OH) by a single bond. A carboxylic acid may be in the form of the protonated carboxylic acid, in the form of a salt of a carboxylic acid (e.g., an ammonium, sodium, or potassium salt), or as a mixture of protonated carboxylic acid and salt of a carboxylic acid. The term carboxylic acid may describe a single chemical species (e.g., oleic acid) or a mixture of carboxylic acids as can be produced, for example, by the hydrolysis of biomass-derived fatty acid esters or triglycerides, diglycerides, monoglycerides, and phospholipids.

The term "fatty acid" as used herein refers to a carboxylic acid (e.g., aliphatic monocarboxylic acid) having C₄ to C₂₈ carbon atoms (most commonly C₁₂ to C₂₄ carbon atoms), which is either saturated or unsaturated. Fatty acids may also be branched or unbranched. Fatty acids may be derived from, or contained in esterified form, in an animal or vegetable fat, oil, or wax. Fatty acids may occur naturally in the form of glycerides in fats and fatty oils or may be obtained by hydrolysis of fats or by synthesis. The term fatty acid may describe a single chemical species or a mixture of fatty acids. In addition, the term fatty acid also encompasses free fatty acids.

The term "fatty alcohol" as used herein refers to an alcohol having an aliphatic chain of C₄ to C₂₂ carbon atoms, which is either saturated or unsaturated.

The term "fatty aldehyde" as used herein refers to an aldehyde having an aliphatic chain of C₄ to C₂₂ carbon atoms, which is either saturated or unsaturated.

The term "fatty amide" as used herein refers to an amide having a long, aliphatic chain of C₄ to C₂₂ carbon atoms, which is either saturated or unsaturated.

The term "fatty ester" as used herein refers to an ester having a long aliphatic chain of C₄ to C₂₂ carbon atoms, which is either saturated or unsaturated.

As used herein, the term "heterologous" with reference to a polynucleotide or polypeptide refers to a polynucleotide or polypeptide that does not naturally occur in a host cell. It is intended that this term includes proteins that are encoded by naturally occurring genes, mutated genes, synthetic genes and/or over-expressed genes.

As used herein, the term "homologous" with reference to a polynucleotide or protein refers to a polynucleotide or protein that occurs naturally in a host cell.

As used herein, the term "end product" refers to any carbon-source derived product which is enzymatically converted from a fermentable substrate. In some embodiments, the end product is an alcohol, such as ethanol or butanol.

As used herein, the term "co-product" refers to a product produced with another product, that is, a jointly produced product. From time to time, as used herein the term "co-product" be used synonymously with the term "by-product."

As used herein, the term "by-product" refers to a secondary product produced during the production of another product.

As used herein, the term "nutrient profile" refers to the nutrient composition of a food or diet.

As used herein, the term "fermenting organism" refers to any microorganism or cell which is suitable for use in fermentation for directly or indirectly producing an end product.

As used herein, the terms "ethanol producer," "ethanol producing microorganism," or "ethanologen" refer to a fermenting organism that is capable of producing ethanol from a fermentable carbon source (e.g., mono- or oligosaccharide).

As used herein, the terms "butanol producer," "butanol producing microorganism," or "butanologen" refer to a fermenting organism that is capable of producing butanol from a fermentable carbon source (e.g., mono- or oligosaccharide).

As used herein, the terms "isobutanol producer," "isobutanol producing microorganism," or "isobutanologen" refer to a fermenting organism that is capable of producing isobutanol from a fermentable carbon source (e.g., mono- or oligosaccharide)

As used herein, the terms "recovered," "isolated," and "separated" with reference to a protein, cell, nucleic acid or amino acid, refers to a protein, cell, nucleic acid or amino acid that is removed from at least one component with which it is naturally associated.

As used herein, the term "derived" encompasses the terms "originated from," "obtained," "obtainable from," and "isolated from." In some embodiments, the term "derived" refers to a polypeptide encoded by a nucleotide sequence that is produced from a cell in which the nucleotide is naturally present or in which the nucleotide has been inserted.

As used herein, the term "yield" refers to the amount of end product produced using the methods of the present invention. In some embodiments, the term refers to the volume of the end product, and in other embodiments, the term refers to the concentration of the end product.

As used herein, the term "COFA" refers to corn oil fatty acids (e.g., fatty acids from hydrolyzing corn oil).

As used herein, the term "FABE" refers to fatty acid butyl esters (e.g., isobutyl esters).

As used herein, the term "FAEE" refers to fatty acid ethyl esters (e.g., ethyl esters).

As used herein, the term "FAME" refers to fatty acid methyl esters (e.g., methyl esters).

As used herein, the term "WS" refers to whole stillage bottoms of a fermentation column.

Alcohols such as ethanol and butanol may be produced by fermentation of sugars. These fermentable sugars may be derived from any biomass source including corn, cane, cellulosic, or lignocellulosic material, and this biomass may be processed, for example, by liquefaction and/or saccharification to form a mash that is fermented by a microorganism such as yeast. During the fermentation process, various process feedstreams are generated and co-products and/or by-products of these streams may be utilized to manufacture products such as animal feed, fuels (e.g., biodiesel), industrial products (e.g., resins, plastics, lubricants) as well as other products for consumer and industrial use.

The present invention provides co-products and/or by-products of an alcohol fermentation process and methods for producing co-products and/or by-products. The present invention provides distillers co-products and methods for producing distillers co-products, including distillers co-products for animal feed, and methods for producing distillers co-products. The distillers co-products for animal feed of the invention can be used as animal feed or can be used as components in animal feed.

In some embodiments, the methods comprise providing an alcohol (e.g., ethanol or butanol) production process with at least one process feedstream, wherein the at least one process feedstream is used to generate a distillers co-products for animal feed having a crude protein content of at least about 20% or at least about 25%. In some embodiments, the methods and compositions of the present invention include one or more process feedstreams of an alcohol production process. In some embodiments, the methods and compositions of the present invention include at least two or at least three process feedstreams of an alcohol (e.g., butanol or ethanol) production process. In some embodiments, a process feedstream is (i) fatty acids, (ii) lipids, (iii) syrup, (iv) distillers grains (DG), (v) distillers grains and solubles (DGS), (vi) solids from a mash before fermentation; (vii) solids from a whole stillage after fermentation, or any combination thereof. In some embodiments, fatty acids may be derived from oil hydrolysis. In some embodiments, the fatty acids are from corn oil hydrolysis. In some embodiments, lipids may be derived from evaporation of thin stillage. In some embodiments, the DG are dried distillers grains (DDG), wet distillers grains (WDG), dried distillers grains and solubles (DDGS), wet distillers grains and solubles (WDGS), or any combination thereof. In some embodiments, the methods and compositions of the present invention include two, three, four, five, six, seven, eight, nine, or more process feedstreams of an alcohol production process. In some embodiments, the process feedstreams are recycled in an alcohol production process.

The systems and processes of the present invention produce distillers co-products having controlled or optimized contents of one or more of protein, fat, fiber, ash, lipid, amino acids, vitamins, and minerals, which can be used as high-value animal feed or can be used to produce high value animal feed. Amino acids include, for example, essential amino acids such as histidine, isoleucine, leucine, lysine, methionine, phenylalanine, threonine, tryptophan, and valine as well as other amine acids such as alanine, arginine, aspartic acid, asparagine, cysteine, glutamic acid, glutamine, glycine, hydroxylysine, hydroxyproline, ornithine, proline, serine, and tyrosine. Minerals include, for example, calcium, chloride, cobalt, copper, fluoride, iodine, iron, magnesium, manganese, phosphorus, potassium, selenium, sodium, sulfur, and zinc. Vitamins include, for example, vitamins A, C, D, E, K, and B (thiamine, riboflavin, niacin, pantothenic acid, biotin, vitamin B6, vitamin B12 and folate).

Distillers co-products may be modified for a particular animal feed or animal feed market based on the selection of a particular process feedstream of an alcohol production process used to produce the distillers co-products. Distillers co-products may be also modified for a particular animal feed or animal feed market based on the selection of particular process feedstreams of an alcohol production process to be combined to produce the distillers co-products. Distillers co-products of the present invention have the economic benefit of allowing for higher inclusion rates of the distillers co-products in animal feed. Also, the production of distillers co-products of the present invention require less energy.

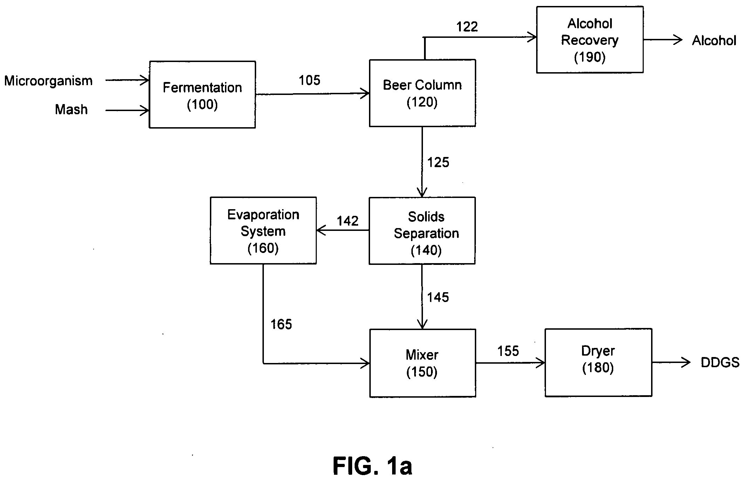

An exemplary system and process of the present invention is described with reference to Figure 1a. Figure 1a illustrates exemplary system and process for fermentation indicating process feedstreams of the present invention. While Figure 1a is described with reference to exemplary process feedstreams, it should be understood that depending on the particular animal feed desired, the process feedstreams combined and unit operations and process settings thereof can be varied from the exemplary process and system of Figure 1a.

Alcohols such as ethanol and butanol may be produced from feedstock derived from biomass. This feedstock may be processed by dry or wet milling and the feedstock may also be subjected to liquefaction and/or saccharification to create a feedstock slurry or mash which comprises fermentable sugars and undissolved solids. For a description of methods and systems for processing biomass for fermentation and separating undissolved solids see, for example, PCT International Publication No. WO 2011/160030.

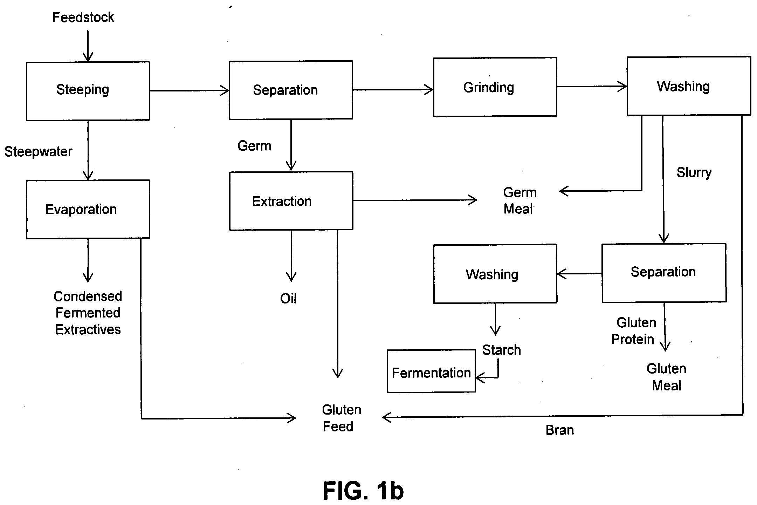

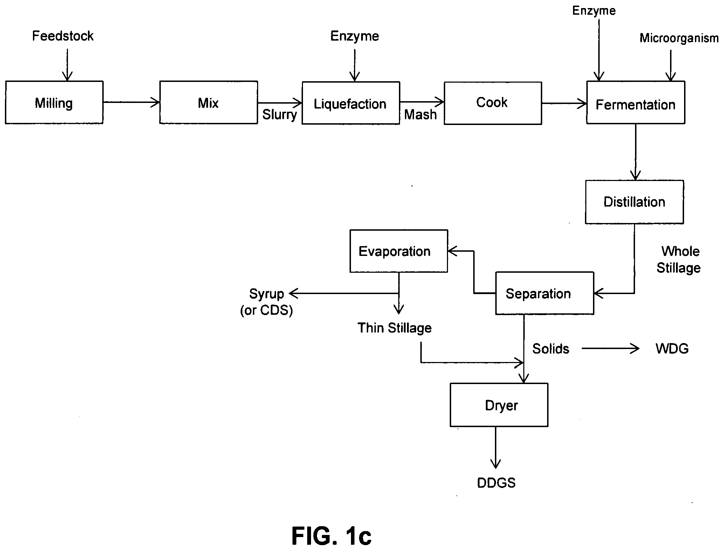

Referring to Figure 1, mash (or feedstock slurry) comprising one or more fermentable carbon sources and one or more microorganisms may be added to fermentation 100 where the mash is fermented by the microorganisms to produce an alcohol such as ethanol or butanol. In some embodiments, the mash is fermented with a microorganism (e.g., yeast) at temperatures in the range of about 15°C to about 40°C, about 20°C to about 38°C, and also about 25°C to about 35°C; at a pH range of about pH 3.0 to about 6.5; also about pH 3.0 to about 6.0; about pH 3.0 to about 5.5, about pH 3.5 to about 5.0 and also about pH 3.5 to about 4.5 for a period of time of about 5 hrs to about 120 hours, preferably about 12 to about 120 and more preferably from about 24 to about 90 hours to produce a product alcohol such as butanol. In some embodiments, the alcohol (e.g., ethanol or butanol) production process comprises fermentation of sugars from corn, barley, wheat, rye, oats, or sugar cane.

Fermentation stream 105 comprising the alcohol may be transferred to a beer column 120. The alcohol may be vaporized within the beer column 120, and the alcohol-rich vaporized stream 122 may be sent to alcohol recovery 190 (e.g., distillation) for further processing of the alcohol. Bottoms stream 125 of the beer column is whole stillage, which contains unfermented solids (e.g., distiller's grain solids), dissolved materials, and water. Bottoms stream 125 may be processed in solids separation 140 and separated into solids 145 (e.g., wet cake) and a liquid stream known as thin stillage 142. Solids separation may be accomplished by a number of means including, but not limited to, centrifugation, filtration, screen separation, hydroclone, or any other means for separating liquids from solids. Thin stillage 142 may be conducted to an evaporation system 160 (e.g., four (4) effect by two (2) body system) for water removal. Examples of evaporation systems are described in U.S. Patent Application Publication No. 2011/0315541, which is incorporated herein in its entirety by reference. Evaporation system 160 incrementally evaporates water from thin stillage 142 to eventually produce syrup 165. In some embodiments, evaporation system 160 evaporates water from thin stillage 142 such that the weight concentration of water in syrup 165 is from about 40% to about 80%, from about 50% to about 70%, or from about 55% to about 65%. In some embodiments, syrup 165 may be combined with wet cake 145 in mixer 150 to produce mixed feed 155 that is dried in dryer 180 to yield DDGS.

As mentioned above, feedstock may be processed by dry or wet milling processes. Wet milling is a multi-step process that separates a biomass (e.g., corn) into its key components (germ, pericarp fiber, starch, and gluten) in order to capture value from each co-product separately. Using corn as a feedstock, this process produces several co-products: starch, gluten feed, gluten meal, and corn oil streams. These streams may be recombined and processed to produce customized products for the feed industry. Referring to Figure 1b, feedstock (e.g., corn) is conducted to steeping tanks where it is soaked, for example, in a sodium dioxide solution for about 30-50 hours at about 120-130°F. Nutrients released into the water may be collected and evaporated to produce condensed fermented extractives (or steep liquor). Germ may be removed from the soaked feedstock and further processed to recover oil and germ meal. After removal of the germ, the remaining portion of feedstock may be processed to remove bran and to produce a starch and gluten slurry. The slurry may be further processed to separate the starch and gluten protein which may be dried to form gluten meal. The starch stream may be further processed via fermentation to produce an alcohol or may be utilized by the food, paper, or textile industries. For example, the starch stream may be used to produce sweeteners. The gluten meal and gluten feed stream which both contain protein, fat, and fiber, may be used in feeds for dairy and beef cattle, poultry, swine, livestock, equine, aquaculture, and domestic pets. Gluten feed may also be used as a carrier for added micronutrients. Gluten meal also contains methionine and xanthophylls which may be used a pigment ingredient in, for example, poultry feeds (e.g., pigment provides egg yolks with yellow pigmentation. Condensed fermented extractives which contains protein, growth factors, B vitamins, and minerals and may be used as a high energy liquid feed ingredient. Condensed extractives may also be used as a pellet binder.

Fractionation removes fiber and germ, which contains a majority of the lipids present in ground whole corn resulting in a fractionated corn that has a higher starch (endosperm) content. Dry fractionation does not separate the germ from fiber and therefore, it is less expensive than wet milling. However, fractionation does not remove the entirety of the fiber or germ, and does not result in total elimination of solids. Furthermore, there is some loss of starch in fractionation.

Dry milling may also be utilized for feedstock processing. Referring to Figure 1c, feedstock may be milled, for example, using a hammermill to generate a meal that may then be mixed with water to form a slurry. The slurry may be subjected to liquefaction by the addition of enzyme such as an amylase to hydrolyze starch to sugars, forming a mash. The mash may heated ("cooked") to inactivate the enzyme and then cooled for addition to fermentation. Cooled mash (i.e., fermentation broth), microorganism, and enzyme such as glucoamylase may be added to fermentation for the production of alcohol (e.g., ethanol or butanol). Following fermentation, the fermentation broth may be conducted to distillation for recovery of the alcohol. The bottoms stream of the distillation column is whole stillage which contains unfermented solids (e.g., distiller's grain solids), dissolved materials, and water may be collected for further processing. The whole stillage may be separated into solids (e.g., wet cake) and thin stillage. Solids separation may be accomplished by a number of means including, but not limited to, centrifugation, filtration, screen separation, hydrocyclone, or any other means for separating liquids from solids. Thin stillage may be conducted to evaporation forming condensed distillers solubles (CDS) or syrup. Thin stillage may comprise soluble nutrients, small grain solids (or fine particles), and microorganisms (e.g., yeast). The solids (wet cake) may be combined with syrup and then dried to form Distillers Dried Grains with Solubles (DDGS). Syrup contains protein, fat, and fiber as well as vitamins and minerals such as phosphorus and potassium; and may be added to animal feeds for its nutritional value and palatability. DDGS contains protein, fat, and fiber; and provides a source of bypass proteins. DDGS may be used in animal feeds for dairy and beef cattle, poultry, swine, livestock, equine, aquaculture, and domestic pets.

As described above, feedstock may be liquefied to produce a feedstock slurry which comprises fermentable sugars and undissolved solids. If the feedstock slurry is fed directly to the fermentor, the undissolved solids may interfere with efficient removal and recovery of the alcohol (e.g., ethanol or butanol) from the fermentor. For example, when liquid-liquid extraction is utilized to extract the alcohol from the fermentation broth, the presence of the undissolved solids may cause system inefficiencies including, but not limited to, decreasing the mass transfer rate of the alcohol to the extractant by interfering with the contact between the extractant and the fermentation broth and reducing the efficiency of recovering and recycling the extractant because at least a portion of the extractant and alcohol becomes "trapped" in the solids which are ultimately removed as DDGS. Thus, in order to avoid and/or minimize these problems, at least a portion of the undissolved solids may be removed from the feedstock slurry prior to the addition of the feedstock slurry to the fermentor. Extraction activity and the efficiency of alcohol production are increased when extraction is performed on a fermentation broth containing an aqueous solution wherein undissolved solids have been removed relative to extraction performed on a fermentation broth containing an aqueous solution wherein undissolved solids have not been removed (see, e.g., PCT International Publication No. WO 2011/160030).

Removal of undissolved solids from the feedstock slurry has several additional benefits. For example, since the undissolved solids are not sent to the fermentation vessel, microorganisms do not contact the undissolved solids. Since the undissolved solids are not exposed to microorganisms as well as extractant, product alcohol, or other by-products of the fermentation, processing of these solids for animal feed may be improved. In addition, removal of undissolved solids prior to fermentation may allow separation and recycle of microorganisms. The ability to recycle the microorganisms may reduce or eliminate the need to grow additional microorganisms for the fermentation process and the need for additional equipment for microbial growth (e.g., propagation tanks).

Separation of the feedstock slurry produces a solid phase (e.g., wet cake) that may be further processed. Wet cake may include a portion of fermentable sugars. As an example of processing the wet cake, wet cake may be washed with water to recover the fermentable sugars present in the wet cake, and the recovered fermentable sugars may be recycled and, for example, used in the liquefaction process. After washing, wet cake may be further processed, for example, to form animal feed.

The process for solids removal may be modified to include discharge of an oil stream from the separation process. For example, if corn is the feedstock, corn oil may also be produced during the preparation of the feedstock. Oil may not be discharged separately from the undissolved solids, and may ultimately be present in the wet cake. When the wet cake is removed via centrifugation or other separation means as described herein, a portion of oil from the feedstock (e.g., corn oil from corn feedstock) may remain with the wet cake. The wet cake may be washed with, for example, additional water in a centrifuge or other separation device. In some embodiments, the oil may be separated from the wet cake and, for example, converted to an extractant for subsequent use in the same or different fermentation process.

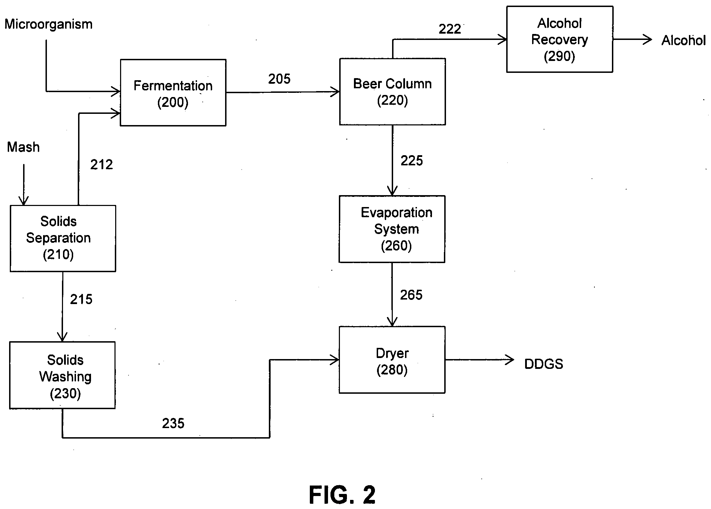

An exemplary system and process of the present invention is described with reference to Figure 2. Feedstock may be liquefied to generate a liquefied mash (or feedstock slurry) comprising undissolved solids and fermentable sugars. Liquefied mash may enter solids separation 210 to form wet cake 215 comprising undissolved solids and a clarified solution of dissolved fermentable sugars (e.g., thin mash) 212. The undissolved solids may be separated from the liquefied mash (or feedstock slurry) by a number of means including, but not limited to, decanter bowl centrifugation, tricanter centrifugation, disk stack centrifugation, filtering centrifugation, decanter centrifugation, filtration, vacuum filtration, beltfilter, pressure filtration, filtration using a screen, screen separation, grating, porous grating, flotation, hydroclone, filter press, screwpress, gravity settler, vortex separator, or combination thereof. Wet cake 215 may be conducted to solids washing 230 to recover fermentable sugars from wet cake 215. Washed wet cake 235 is formed and the total wash liquids generated in solids washing 230 may be recycled and, for example, used in the liquefaction process. Washed wet cake 235 may be combined with syrup 265 in dryer 280 to produce DDGS.

Thin mash 212 and microorganisms may be added to fermentation 200 where the mash is fermented by the microorganism to produce fermentation stream 205 comprising an alcohol such as ethanol or butanol. Fermentation stream 205 may be conducted to beer column 220 to produce alcohol-rich stream 222 and bottoms stream 225. Alcohol-rich stream 222 may be sent to alcohol recovery 290 for recovery of the product alcohol. Bottoms stream 225 comprising thin stillage, with most of the solids removed prior to fermentation, may be concentrated by evaporation via evaporation system 260 to form syrup 265.

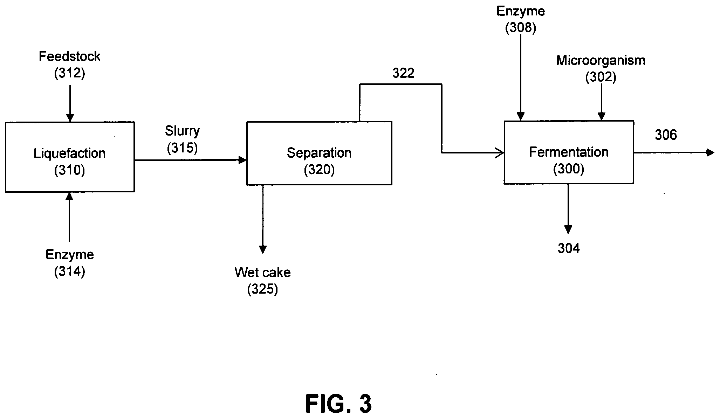

As described above, feedstock may be processed to produce a feedstock slurry which comprises fermentable sugars and undissolved solids. Figures 3 to 5 provide exemplary systems and processes that may be utilized to process feedstock. In some embodiments, as shown, for example, in Figure 3, the system includes a liquefaction vessel 310 configured to liquefy a feedstock to create a feedstock slurry. In particular, feedstock 312 can be introduced to an inlet in liquefaction vessel 310. Feedstock 312 can be any suitable biomass material known in the industry including, but not limited to, rye, wheat, cane, or corn, that contains a fermentable carbon source such as starch.

The process of liquefying feedstock involves hydrolysis of starch in feedstock 312 into water-soluble sugars. Any known liquefying processes, as well as the corresponding liquefaction vessel, normally utilized by the industry can be used including, but not limited to, the acid process, the acid-enzyme process, or the enzyme process. Such processes can be used alone or in combination. In some embodiments, the enzyme process can be utilized and an appropriate enzyme 314, for example, alpha-amylase, is introduced to an inlet in liquefaction vessel 310. Water can also be introduced to the liquefaction vessel 310.

The process of liquefying feedstock 312 creates a feedstock slurry 315 that includes sugar (e.g., fermentable carbon) and undissolved solids from the feedstock or biomass. The undissolved solids are non-fermentable portions of feedstock 312. In some embodiments, feedstock 312 can be corn, such as dry milled, unfractionated corn kernels, and the undissolved particles can include germ, fiber, and gluten. Feedstock slurry 315 can be discharged from an outlet of liquefaction vessel 310. In some embodiments, feedstock 312 is corn or corn kernels and feedstock slurry 315 is a corn mash slurry.

Separation 320 configured to remove the undissolved solids from feedstock slurry 315 has an inlet for receiving feedstock slurry 315. Separation 320 agitates or spins feedstock slurry 315 to create a liquid phase or aqueous solution 322 and a solid phase or wet cake 325.

Aqueous solution 322 can include sugar, for example, in the form of oligosaccharides, and water. Aqueous solution can comprise at least about 10% by weight oligosaccharides, at least about 20% by weight of oligosaccharides, or at least about 30% by weight of oligosaccharides. Aqueous solution 322 can be discharged out an outlet located near the top of separation 320. Aqueous solution can have a viscosity of less than about 20 centipoise, or less than about 15 centipoise, or less than about 10 centipoise, or less than about 5 centipoise. The aqueous solution can comprise less than about 20 g/L of monomeric glucose, or less than about 10 g/L, or less than about 5 g/L of monomeric glucose. Suitable methodology to determine the amount of monomeric glucose is well known in the art. Such suitable methods known in the art include HPLC.

Wet cake 325 can include the undissolved solids. Wet cake 325 can be discharged from an outlet located near the bottom of separation 320. Wet cake 325 can also include a portion of the sugar and water. Wet cake 325 can be washed with additional water in separation 320 once aqueous solution 322 has been discharged from separation 320. Alternatively, wet cake 325 can be washed with additional water in another separation device (e.g., centrifuge). Washing wet cake 325 will recover the sugar or sugar source (e.g., oligosaccharides) present in the wet cake, and the recovered sugar and water may be recycled to liquefaction 310. After washing, wet cake 325 may be further processed to form DDGS through any suitable known process. The formation of the DDGS from wet cake 325 formed in separation 320 has several benefits. Since the undissolved solids do not go to the fermentor, extractant, and/or alcohol are not trapped in the DDGS, DDGS is not subjected to the conditions of the fermentor, and DDGS does not contact the microorganisms present in the fermentor. All these effects provide benefits to subsequent processing and selling of DDGS, for example as animal feed.

Separation 320 can be any conventional centrifuge utilized in the industry, including, for example, a decanter bowl centrifuge, tricanter centrifuge, disk stack centrifuge, filtering centrifuge, or decanter centrifuge. In some embodiments, removal of the undissolved solids from feedstock slurry 315 can be accomplished by filtration, vacuum filtration, beltfilter, pressure filtration, filtration using a screen, screen separation, grates or grating, porous grating, flotation, hydroclone, filter press, screwpress, gravity settler, vortex separator, or any method that can be used to separate solids from liquids.

If corn is used as feedstock, undissolved solids can be removed from corn mash to form two product streams, for example, an aqueous solution of oligosaccharides which contains a lower concentration of solids as compared to corn mash and a wet cake which contains a higher concentration of solids as compared to corn mash. In addition, a third stream containing corn oil can be generated if, for example, a tricanter centrifuge is utilized for solids removal from corn mash. A tricanter centrifuge can be used for three-phase separation such as the separation of two liquid phases (e.g., aqueous stream and oil stream) and a solid phase (e.g., solids) (see, e.g., Flottweg Tricanter®, Flottweg AG, Vilsibiburg, Germany; Tricanter® Oil Separation System, ICM, Inc., Colwich, KS;). The two liquid phases may be separated and decanted from the bowl via two discharge systems to prevent cross contamination and the solids phase may be removed via a separate discharge system. As such, a number of product streams can be generated by using different separation techniques or a combination thereof.

Fermentation 300 configured to ferment aqueous solution 322 to produce an alcohol has an inlet for receiving aqueous solution 322. Fermentation 300 can include a fermentation broth. Microorganism 302 may be introduced to fermentation 300 and included in the fermentation broth. Microorganism 302 consumes the sugar in aqueous solution 322. In some embodiments, microorganism 302 consumes the sugar in aqueous solution 322 and produces an alcohol such as ethanol or butanol. Stream 306 comprising the alcohol may be discharged from fermentation 300 and processed further for recovery of the alcohol.

In some embodiments, simultaneous saccharification and fermentation (SSF) can occur inside fermentation 300. Any known saccharification process normally utilized by the industry can be used including, but not limited to, the acid process, the acid-enzyme process, or the enzyme process. In some embodiments, enzyme 308 such as glucoamylase, can be introduced to an inlet in fermentation 300 in order to catalyze the breakdown of sugars in the form of oligosaccharides present in aqueous solution 322 into monosaccharides.

In some embodiments, fermentation broth 304 can be discharged from an outlet in fermentation 300. The discharged fermentation broth 304 can include microorganism 302 such as a yeast. Microorganism 302 can be easily separated from the fermentation broth 304 using any suitable separation device, for example, a centrifuge. Microorganism 302 can then be recycled to fermentation 300 which over time can increase the production rate of the alcohol, thereby resulting in an increase in the efficiency of alcohol production.

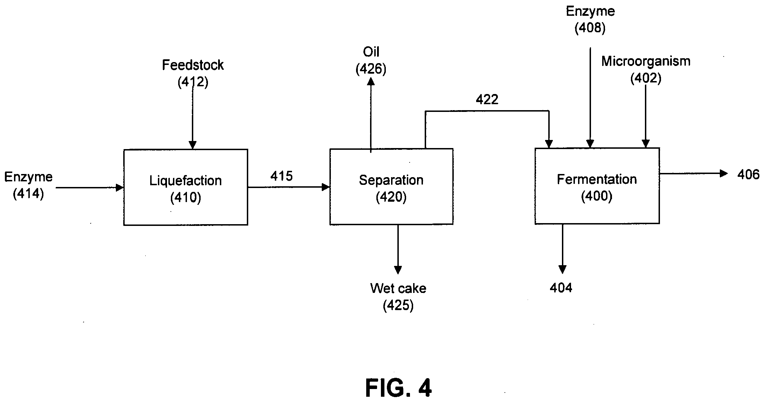

In some embodiments, as shown, for example, in Figure 4, the systems and processes of the present invention can include discharging an oil 426 from an outlet of separation 420. Figure 4 is identical to Figure 3, except for oil stream 426 exiting separation 420 and therefore will not be described in detail again.

Feedstock slurry 415 is separated into a first liquid phase or aqueous solution 422 containing the fermentable sugar, a solid phase or wet cake 425 containing the undissolved solid, and a second liquid phase containing oil 426 which can exit separation 420. In some embodiments, feedstock 412 is corn and oil 426 is corn oil. Any suitable separation device can be used to discharge aqueous solution 422, wet cake 425, and oil 426, for example, a tricanter centrifuge. In some embodiments, a portion of the oil from feedstock 412 such as corn oil when the feedstock is corn, remains in wet cake 425. In some embodiments, wet cake 425 includes corn oil in an amount of less than about 20% by weight of dry solids content of wet cake 425.

In some embodiments, when feedstock 412 (e.g., corn) and corn oil 426 are removed from separation 420, the fermentation broth in fermentation 400 includes a reduced amount of corn oil. For example, the fermentation broth, substantially free of undissolved solid, can include an alcohol portion (e.g., butanol) and an oil portion (e.g., corn oil) in a ratio of at least about 4:1 on a weight basis, at least about 3:1 on a weight basis, or at least about 2:1 on a weight basis. The corn oil can contain, for example, at least 15% by weight of free fatty acids or at least 16.7% by weight of free fatty acids.

In some embodiments, separation 420 produces a product profile including a layer of undissolved solids, a layer of oil (e.g., corn oil), and a supernatant layer including the fermentable sugars. The ratio of fermentable sugars in the supernatant layer to undissolved solids in the undissolved solids layer on a weight base can be in a range from about 2:1 to about 5:1; the ratio of fermentable sugars in the supernatant layer to corn oil in the corn oil layer on a weight basis can be in a range from about 10:1 to about 50:1; and/or the ratio of undissolved solids in the undissolved solids layer to corn oil in the corn oil layer on a weight basis can be in a range from about 2:1 to about 25:1.

If oil 426 is not discharged separately it can be removed with wet cake 425. When wet cake 425 is removed via separation 420, in some embodiments, a portion of the oil from feedstock 412, such as corn oil when the feedstock is corn, remains in wet cake 425. Wet cake 425 can be washed with additional water in separation 420 once aqueous solution 422 has been discharged from separation 420. Washing wet cake 425 will recover the sugar (e.g., oligosaccharides) present in the wet cake and the recovered sugar and water can be recycled to the liquefaction 410. After washing, wet cake 425 can be combined with solubles and then dried to form DDGS through any suitable known process. The formation of the DDGS from wet cake 425 formed in separation 420 has several benefits. In some embodiments, oil 426 is not discharged separately from wet cake 425, but rather oil 426 is included as part of wet cake 425 and is ultimately present in the DDGS. In such instances, the oil can be separated from the DDGS and converted to an extractant (e.g., an extractant for ISPR) for subsequent use in the same or different alcohol fermentation process.

Oil 426 can be separated from DDGS using any suitable known process including, for example, a solvent extraction process. In some embodiments of the invention, DDGS are loaded into an extraction vessel and washed with a solvent such as hexane to remove oil 426. Other solvents that can be utilized include, for example, isobutanol, isohexane, ethanol, petroleum distillates such as petroleum ether, or mixtures thereof. After oil 426 extraction, DDGS can be treated to remove any residual solvent. For example, DDGS can be heated to vaporize any residual solvent using any method known in the art. Following solvent removal, DDGS can be subjected to a drying process to remove any residual water. The processed DDGS can be used as a feed supplement for animals such as poultry, livestock, ruminant, cattle, dairy animal, swine, goat, sheep, aquaculture (e.g., salmon, catfish, trout, shrimp), equine, and domestic pets.

After extraction from DDGS, the resulting oil 426 and solvent mixture can be collected for separation of oil 426 from the solvent. In some embodiments, the oil 426/solvent mixture can be processed by evaporation whereby the solvent is evaporated and can be collected and recycled. The recovered oil can be converted to an extractant (e.g., an extractant for ISPR) for subsequent use in the same or different alcohol fermentation process.

Removal of the oil component of the feedstock is advantageous to alcohol production because oil present in the fermentor can break down into fatty acids and glycerin. The glycerin can accumulate in the water and reduce the amount of water that is available for recycling throughout the system. Thus, removal of the oil component of the feedstock increases the efficiency of alcohol production by increasing the amount of water that can be recycled through the system.

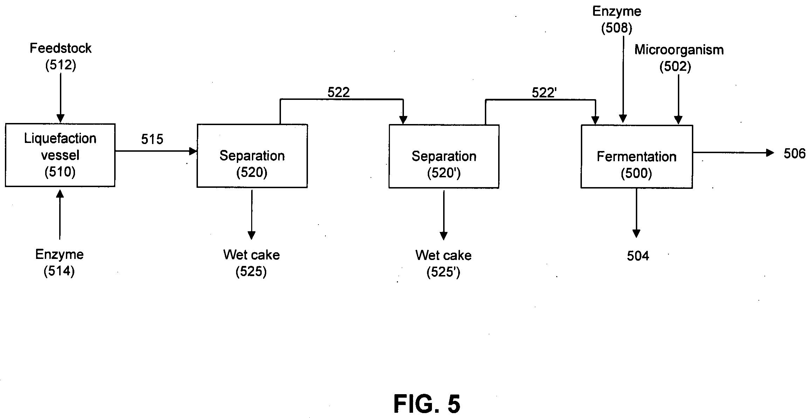

In some embodiments, as shown, for example, in Figure 5, the systems and processes of the present invention can include a series of two or more separation devices (e.g., centrifuges). Figure 5 is identical to Figure 3, except for the addition of a second separation device 520' and therefore will not be described in detail again.

Aqueous solution 522 discharged from separation 520 can be received in an inlet of separation 520'. Separation 520' can be identical to separation 520 and can operate in the same manner. Separation 520' can remove undissolved solids not separated from aqueous solution 522 in separation 520 to create (i) an aqueous stream 522' similar to aqueous stream 522, but containing reduced amounts of undissolved solids in comparison to aqueous stream 522 and (ii) a wet cake 525' similar to wet cake 525. Aqueous stream 522' can then be introduced to fermentation 500. In some embodiments, there can be one or more additional separation devices after separation 520'. Microorganism 502 and enzyme 508 can be added to fermentation 500 producing alcohol stream 506 which may be further processed for recovery of the alcohol.

Fermentation broth 504 can be discharged from fermentation 500. Discharged fermentation broth 504 can include microorganism 502. Microorganism 502 can be separated from the fermentation broth 504 using any suitable separation device, for example, a centrifuge. Microorganism 502 can then be recycled to fermentation 500 which over time can increase the production rate of the alcohol, thereby resulting in an increase in the efficiency of alcohol production.

If corn is used as the source of the milled grain, corn oil can be separated from the process streams at any of several points. For example, a centrifuge can be operated to produce a corn oil stream following filtration of the cooked mash. Intermediate concentration syrup or final syrup can be centrifuged to produce a corn oil stream.

In some embodiment of the methods of the invention, the material discharged from the fermentor can be processed in a separation system that involves devices such as a centrifuge, settler, hydrocyclone, etc., and combinations thereof to effect the recovery of live yeast in a concentrated form that can be recycled for reuse in a subsequent fermentation batch either directly or after some re-conditioning. This separation system can also produce an organic stream that comprises fatty esters (e.g., isobutyl fatty esters) and an alcohol (e.g., butanol) produced from the fermentation and an aqueous stream containing only trace levels of immiscible organics. This aqueous stream can be used either before or after it is stripped of the alcohol (e.g., butanol) content to re-pulp and pump the low starch solids that was separated and washed from liquefied mash. In some embodiments, the multi-phase material can leave the bottom of column and can be processed in a separation system as described above. The concentrated solids can be redispersed in the aqueous stream and this combined stream can be used to re-pulp and pump the low starch solids that were separated and washed from liquefied mash.

Alcohols such as butanol may be recovered from fermentation broth by extractive fermentation. In general, the fermentation broth is contacted with an extractant forming a biphasic or two-phase mixture comprising an alcohol-containing organic phase and an aqueous phase. The extraction process may be conducted within the fermentation vessel (i.e., in situ product removal) or downstream of the fermentation vessel. In situ product removal (ISPR) may be carried out in a batch mode, fed-batch mode, or continuous mode. Methods for producing and recovering alcohols from a fermentation broth using extractive fermentation are described in U.S. Patent Application Publication No. 2009/0305370; U.S. Patent Application Publication No. 2010/0221802; U.S. Patent Application Publication No. 2011/0097773; U.S. Patent Application Publication No. 2011/0312044; and U.S. Patent Application Publication No. 2011/0312043.

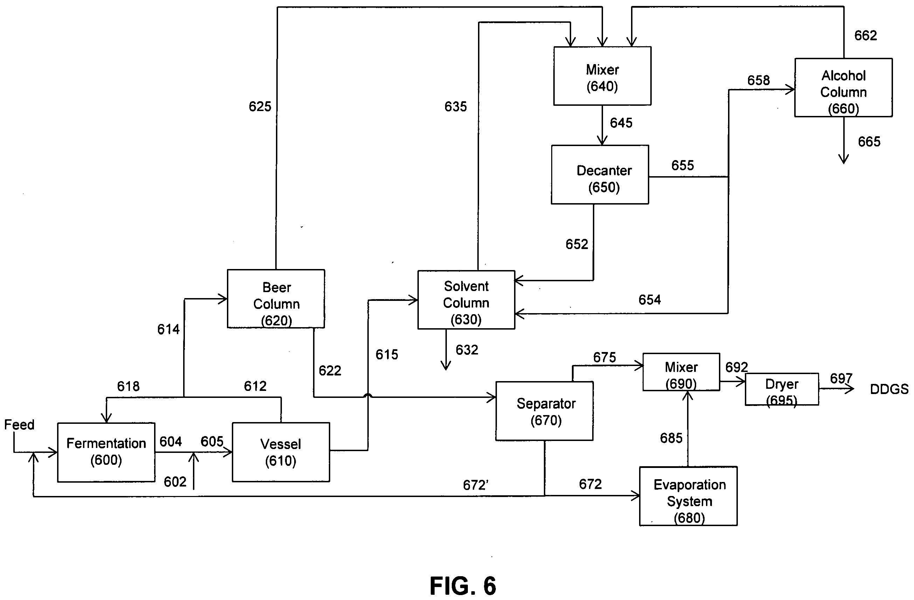

An example of extractive fermentation is illustrated in Figure 6. Fermentation broth 604 may be removed from fermentation 600 on a continuous or periodic basis, and extractant 602 is added to fermentation broth 604 to obtain to a biphasic mixture 605 obtained by contacting the fermentation broth with extractant 602. The biphasic mixture is introduced in a vessel 610, in which separation of the aqueous and organic phases is performed to produce an alcohol-containing organic phase 615 and an aqueous phase 612. Extractant 602 may be a water-immiscible organic solvent or solvent mixture.

The extraction of the alcohol product by the extractant may be done with or without the removal of the microorganism from the fermentation broth. The microorganism may be removed from the fermentation broth by means known in the art including, but not limited to, filtration or centrifugation. In some embodiments, extractant 602 may be added to fermentation broth 604 in a separate vessel prior to introduction to vessel 610. Alternatively, extractant 602 may be contacted with fermentation broth 604 after introduction into vessel 610 to obtain biphasic mixture 605 which is then separated into the organic and aqueous phases. Alcohol-containing organic phase 615 may be separated from the aqueous phase 612 of the biphasic fermentation broth using methods known in the art, including but not limited to, siphoning, decantation, centrifugation, using a gravity settler, membrane-assisted phase splitting, and the like.

As illustrated in Figure 6, the product alcohol may be extracted from the fermentation broth downstream of fermentation 600. Alternatively, the two-phase extractive fermentation method may be carried out *in situ*, in a batch mode or a continuous mode in the fermentor. For *in situ* extractive fermentation, the extractant may contact the fermentation broth at the start of the fermentation forming a biphasic fermentation broth. Alternatively, the extractant may contact the fermentation broth after the microorganism has achieved a desired amount of growth, which can be determined by measuring the optical density of the culture. Further, the extractant may contact the fermentation broth at a time at which the alcohol level in the fermentation broth reaches a preselected level, for example, before the alcohol concentration reaches a toxic level. After contacting the fermentation broth with the extractant, the product alcohol partitions into the extractant, decreasing the concentration in the aqueous phase containing the microorganism, thereby limiting the exposure of the production microorganism to the inhibitory product alcohol. The volume of the extractant to be used depends on a number of factors, including the volume of the fermentation broth, the size of the fermentor, the partition coefficient of the extractant for the product alcohol, and the fermentation mode chosen, as described below.

In a continuous mode of *in situ* extractive fermentation, in one embodiment, extractant 602 may be introduced into fermentation 600 to obtain the biphasic mixture 605 therein, with the alcohol-containing organic-phase stream 615 and aqueous phase stream 612 exiting directly from fermentation 600. In another embodiment, the mixture of the fermentation broth and the alcohol-containing extractant is removed from fermentation 600, and the alcohol-containing organic phase is then separated from the aqueous phase. The fermentation broth may be recycled to fermentation 600 or may be replaced with fresh broth. Then, the extractant is treated to recover the product alcohol, and the extractant may then be recycled back into fermentation 600 for further extraction of the product alcohol. Alternatively, fresh extractant may be continuously added to fermentation 600 to replace the removed extractant. In a batchwise mode of *in situ* extractive fermentation, a volume of extractant is added to the fermentor to form a two-phase mixture and the extractant is not removed during the process.

After separation of the fermentation broth from the extractant by means described above, the fermentation broth may be recycled to fermentation 600, discarded, or treated for the removal of any remaining product alcohol. After separation of the fermentation broth from the extractant, the aqueous phase 612 is split into a feed stream 614 and a recycle stream 618. Recycle stream 618 returns a portion of the fermentation broth to fermentation 600. Similarly, if the microorganism was removed from the fermentation broth prior to contact with the extractant, the isolated microorganism may also be recycled to fermentation 600. Feed stream 614 is introduced into a beer column 620 for recovery of product alcohol.

After extracting the product alcohol from the fermentation broth, the product alcohol is recovered from alcohol-containing organic phase 615. Alcohol-containing organic phase 615 typically comprises the extractant, water, product alcohol, and optionally a non-condensable gas. Alcohol-containing organic phase 615 may optionally further comprise fermentation by-products having sufficient solubility to partition into the extractant phase.

Recovery of the product alcohol from the alcohol-containing organic phase may be done using methods known in the art, including but not limited to, distillation, adsorption by resins, separation by molecular sieves, pervaporation, and the like. The exemplary system of Figure 6 incorporates a combination of distillation and decantation to recover the product alcohol from alcohol-containing organic phase 615. The distillation to recover the product alcohol from the alcohol-containing organic phase 615 involves the use of at least two distillation columns: a solvent column 630 and an alcohol (e.g., butanol) column 660. Solvent column 630, in combination with decantation, effects a separation of any non-condensable gas, such as carbon dioxide, and product alcohol from the extractant, and water.

Alcohol-containing organic phase 615 is distilled in solvent column 630 to provide an product alcohol-rich vaporous overhead stream 635 comprising water, product alcohol, and non-condensable gas if present in the feed, and a solvent-rich liquid bottoms stream 632 comprising the extractant and water and being substantially free of product alcohol. In some embodiments, recovered extractant stream 632 may be recycled to the extractive fermentation process. For example, recovered extractant stream 632 may be used as the extractant 602 that is contacted with fermentation broth 604.

Vaporous overhead stream 635 may include up to about 65 wt% product alcohol and at minimum about 30 wt% water. In some embodiments, vaporous overhead stream includes from about 65 wt% product alcohol and at minimum about 32 wt% water, from about 60 wt% product alcohol and at minimum about 35 wt% water in another embodiment, from about 55 wt% product alcohol and at minimum about 40 wt% water in another embodiment, and from about 50 wt% to about 55 wt% product alcohol and from about 45 wt% to about 50 wt% water in other embodiment. In some embodiments, the amount of extractant in vaporous overhead stream 635 is less than 2 wt%. In some embodiments, vaporous overhead stream 635 may be cooled and condensed in a condenser and combined in a mixer 640 with condensed vaporous overhead streams 625 and 662 from beer column 620 and column 660, respectively. The combined stream 645 may be decanted in a decanter 650 into an alcohol-rich liquid phase and an alcohol-poor liquid aqueous phase. For example, the liquid alcohol phase may include less than about 30 wt% water, or from about 20 to about 30 wt% water, or from about 16 to about 30 wt% water, or from about 10 to about 20 wt% water, and may further comprise less than about 0.001 weight percent of residual extractant which comes overhead in solvent column 630. The liquid aqueous phase may include less than about 10 wt% product alcohol, or in some embodiments, from about 3 to about 10 wt% product alcohol. All or part of the liquid aqueous phase from decanter 650 may be returned to solvent column 630 as a reflux stream 652. A stream 655 of the alcohol-rich liquid phase from decanter 650 may be split, with a portion returned to solvent column 630 as a reflux stream 654 and the remainder portion 658 fed to column 660. Column 660 effects a separation of product alcohol and water and provides a product alcohol bottoms stream 665 which is substantially 100 wt% product alcohol and substantially free of water. Vaporous overhead stream 662 comprises product alcohol and water, for example about 67 wt% product alcohol and about 33 wt% water, for example 60 wt% product alcohol and about 40 wt% water, or for example 55 wt% product alcohol and about 45 wt% water. In some embodiments, vaporous overhead stream 662 may be condensed in a condenser and return to decanter 650 via mixer 640.

After separation of the fermentation broth from the extractant, the aqueous phase feed stream 614 is introduced into beer column 620 to provide a product alcohol-rich vaporous overhead stream 625 comprising water, product alcohol, and non-condensable gas if present in the feed, and a product alcohol-poor beer bottoms liquid stream 622. Beer bottoms stream 622 comprises by-products such as distiller's grains and thin stillage.

Since the beer bottom by-products have value as feedstock, it is desirable to further process all or part of these by-products into one or more of DDG, WDG, Distillers Dried Solubles (DDS), CDS, DDGS, corn oil, and/or COFA rather than discarding the beer bottoms as waste. In the embodiment of Figure 6, beer bottoms stream 622 is further processed to produce DDGS 697. To that end, beer bottoms stream 622 is introduced into a separator 670, which can be a mechanical separator such as a centrifuge or filter press, for separating the grain solids 675 of the beer bottoms from thin stillage which primarily includes water. A portion 672' of the thin stillage may be recycled to the feed introduced into fermentation 600. The remainder thin stillage 672 may be concentrated into syrup 685 by evaporating a substantial amount of water therefrom in evaporation system 680. In some embodiments, evaporation system 680 achieves evaporation of the water from thin stillage 672 such that the weight concentration of water in syrup 685 is from about 40% to about 65%. In some embodiments, the weight concentration water in syrup 685 is from about 45% to about 60%. In some embodiments, the weight concentration of water in thin stillage 672 is from about 85% to about 95%, and in some embodiments, the weight concentration of water in thin stillage 672 is about 90%. Syrup 685 may then be combined with grain solids 675 in a mixer 690, and the combined stream 692 of grains and syrup can then be dried in a dryer 695 to produce DDGS 697.

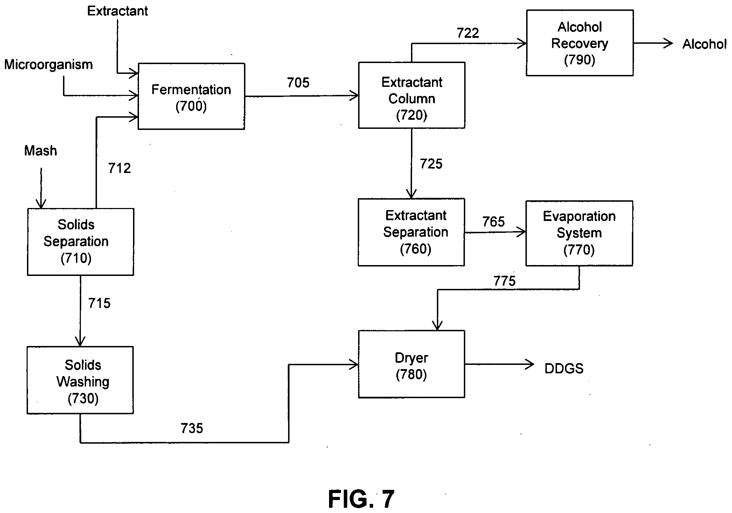

Figure 7 illustrates another embodiment of the systems and processes of the invention. Feedstock may be liquefied to generate a liquefied mash (or feedstock slurry) comprising undissolved solids and fermentable sugars. Liquefied mash may enter solids separation 710 to form wet cake 715 comprising undissolved solids and a clarified solution of dissolved fermentable sugars (e.g., thin mash) 712. The undissolved solids may be separated from the liquefied mash (or feedstock slurry) by a number of means including, but not limited to, decanter bowl centrifugation, tricanter centrifugation, disk stack centrifugation, filtering centrifugation, decanter centrifugation, filtration, vacuum filtration, beltfilter, pressure filtration, filtration using a screen, screen separation, grating, porous grating, flotation, hydroclone, filter press, screwpress, gravity settler, vortex separator, or combination thereof. Wet cake 715 may be conducted to solids washing 730 to recover fermentable sugars from wet cake 715. Washed wet cake 735 is formed and may be conducted to dryer 780 to produce DDGS.

Thin mash 712, microorganisms, and extractant may be added to fermentation 700 where the mash is fermented by the microorganism to produce biphasic stream 705. Biphasic stream 705 may be conducted to extractant column 720 to produce vaporous stream 722 and bottoms stream 725. Vaporous stream 722 may be sent to alcohol recovery 790 for recovery of the product alcohol. Bottoms stream 725 may be conducted to extractant separation 760 to separate the stream into thin stillage 765 and extractant. In some embodiments, the recovered extractant may be recycled to fermentation 700. Thin stillage 308, with most of the solids removed prior to fermentation, may be concentrated by evaporation via evaporation system 770 to form syrup 775. Syrup 775 may be combined with wet cake 735 in dryer 780 to produce DDGS.

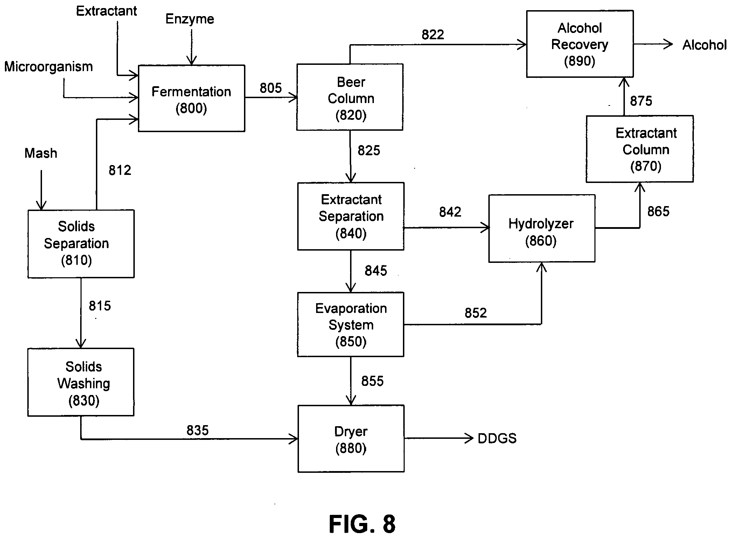

Figure 8 illustrates a modification of the process shown in Figure 7 where the extractant is a fatty acid derived from an oil (e.g., corn oil). An esterification catalyst may be provided to promote chemical reaction between the fatty acid and product alcohol (e.g., butanol) to form fatty acid ester. An enzyme (e.g., lipase) may be added with the microorganism, thin mash 812, and extractant in fermentation 800 so that a portion of the product alcohol produced during fermentation may be chemically sequestered as fatty acid ester (e.g., fatty acid butyl ester). Fermentation discharge 805 may contain some product alcohol which may be recovered in beer column 820. Biphasic bottoms stream 825 may contain the ester product and this may be separated in extractant separation 840 to form fatty ester stream 842 and thin stillage 845. In some embodiments, thin stillage 845 may comprise the ester and when evaporated in evaporation system 850, the ester may be recovered as stream 852. The combined fatty ester contained in streams 842 and 852 may be chemically treated in hydrolyzer 860 to convert the fatty ester to fatty acid and product alcohol. Biphasic stream 865 from hydrolyzer 860 may be conducted to extractant column 870 to recover product alcohol. The bottoms of extractant column 870 comprises fatty acid that may be recycled to fermentation 800.

Extractants that may be utilized for the processes described herein include, for example, organic solvents. In some embodiments, extractants may be water-immiscible organic solvents. For example, extractants such as C₇ to C₂₂ fatty alcohols, C₇ to C₂₂ fatty acids, esters of C₇ to C₂₂ fatty acids, C₇ to C₂₂ fatty aldehydes, C₇ to C₂₂ fatty amides, and mixtures thereof may be used utilized for the processes described herein. In some embodiments, extractants may be selected from C₁₂ to C₂₂ fatty alcohols, C₁₂ to C₂₂ fatty acids, esters of C₁₂ to C₂₂ fatty acids, C₁₂ to C₂₂ fatty aldehydes, C₁₂ to C₂₂ fatty amides, and mixtures thereof. In some embodiments, extractants may include a first extractant selected from C₁₂ to C₂₂ fatty alcohols, C₁₂ to C₂₂ fatty acids, esters of C₁₂ to C₂₂ fatty acids, C₁₂ to C₂₂ fatty aldehydes, C₁₂ to C₂₂ fatty amides, and mixtures thereof; and a second extractant selected from C₇ to C₁₁ fatty alcohols, C₇ to C₁₁ fatty acids, esters of C₇ to C₁₁ fatty acids, C₇ to C₁₁ fatty aldehydes, and mixtures thereof. In some embodiments, extractants may be carboxylic acids. Additional examples of extractants include oleyl alcohol, behenyl alcohol, cetyl alcohol, lauryl alcohol, myristyl alcohol, stearyl alcohol, oleic acid, lauric acid, myristic acid, stearic acid, methyl myristate, methyl oleate, lauric aldehyde, 1-nonanol, 1-decanol, 1-undecanol, 2-undecanol, 1-nonanal, and mixtures thereof.

Methods for producing and recovering a product alcohol from a fermentation broth using extractive fermentation are described in U.S. Patent Application Publication No. 2009/0305370; U.S. Patent Application Publication No. 2010/0221802; U.S. Patent Application Publication No. 2011/0097773; U.S. Patent Application Publication No. 2011/0312044; and U.S. Patent Application Publication No. 2011/0312043; U.S. Patent Application Publication No. 2010/0143992; U.S. Patent Application Publication No. 2010/0143993; U.S. Patent Application Publication No. 2010/0143994; and U.S. Patent Application Publication No. 2010/0143995; the entire contents of each are herein incorporated by reference.

Using extractive fermentation as described herein, extractant (e.g., fatty acids, COFA, fatty acid esters) or oil may be present in the distillers co-products. In some embodiments, extractant and/oil may be removed from the distillers co-products. For example, extractant and/oil may be removed from distillers co-products using a mechanical means such as a screwpress or centrifuge or chemical means such as extraction with hexane or an alcohol (e.g., butanol), treatment with hydrogen peroxide, ion exchange, or distillation. Removal of extractant and/oil may improve the quality of the distillers co-products by reducing the fat content and increasing the protein content of the distillers co-products.

The various streams generated during the processes and systems described herein may be further processed to generate co-products such as DDGS or fatty acid esters. Co-products may be formed by recovering the by-products from a stream or by combining and mixing several streams. For example, fatty acid esters may be recovered by using a solvent to extract the esters from the thin stillage stream or the wet cake. A solvent-based extraction system is described in U.S. Patent Application Publication No. 2010/0092603, the teachings of which are incorporated by reference herein.

In some embodiments of solvent extraction of fatty acid esters, solids can be separated from whole stillage ("separated solids") since that stream would contain the largest portion, by far, of fatty acid esters in uncombined by-product streams. These separated solids can then be fed into an extractor and washed with solvent. In some embodiments, the separated solids are turned at least once in order to ensure that all sides of the separated solids are washed with solvent. After washing, the resulting mixture of lipid and solvent, known as miscella, is collected for separation of the extracted lipid from the solvent. For example, the resulting mixture of lipid and solvent can be deposited to a separator for further processing. During the extraction process, as the solvent washes over the separated solids, the solvent not only brings lipid into solution, but it collects fine, solid particles. These "fines" are generally undesirable impurities in the miscella and in some embodiments, the miscella can be discharged from the extractor or separator through a device that separates or scrubs the fines from the miscella.

In order to separate the lipid and the solvent contained in the miscella, the miscella can be subjected to a distillation step. In this step, the miscella can, for example, be processed through an evaporator which heats the miscella to a temperature that is high enough to cause vaporization of the solvent, but is not sufficiently high to adversely affect or vaporize the extracted lipid. As the solvent evaporates, it can be collected, for example, in a condenser, and recycled for future use. Separation of the solvent from the miscella results in a stock of crude lipid which can be further processed to separate water, fatty acid esters (e.g., fatty acid isobutyl esters), fatty acids, and triglycerides.

After extraction of the lipids, the solids can be conveyed out of the extractor and subjected to a stripping process that removes residual solvent. Recovery of residual solvent is important to process economics. In some embodiments, the wet solids can be conveyed in a vapor tight environment to preserve and collect solvent that transiently evaporates from the wet solids as it is conveyed into the desolventizer. As the solids enter the desolventizer, they can be heated to vaporize and remove the residual solvent. In order to heat the solids, the desolventizer can include a mechanism for distributing the solids over one or more trays, and the solids can be heated directly, such as through direct contact with heated air or steam, or indirectly, such as by heating the tray carrying the solids. In order to facilitate transfer of the solids from one tray to another, the trays carrying the solids can include openings that allow the solids to pass from one tray to the next. From the desolventizer, the solids can be conveyed to, optionally, a mixer where the solids are mixed with other by-products before being conveyed into a dryer. In this example, the solids are fed to a desolventizer where the solids are contacted by steam. In some embodiments, the flows of steam and solids in the desolventizer can be countercurrent. The solids can then exit the desolventizer and can be fed to a dryer or optionally a mixer where various by-products can be mixed. Vapor exiting the desolventizer can be condensed and optionally mixed with miscella and then fed to a decanter. The water-rich phase exiting the decanter can be fed to a distillation column where hexane is removed from the water-rich stream. In some embodiments, the hexane-depleted water rich stream exits the bottom of the distillation column and can be recycled back to the fermentation process, for example, it can be used to slurry the ground corn solids. In some embodiments, the overhead and bottom products can be recycled to the fermentation process. For example, the lipid-rich bottoms can be added to the feed of a hydrolyzer. The overheads can be, for example, condensed and fed to a decanter. The hexane rich stream exiting this decanter can optionally be used as part of the solvent feed to the extractor. The water-rich phase exiting this decanter can be fed to the column that strips hexane out of water. As one skilled in the art can appreciate, the methods of the present invention can be modified in a variety of ways to optimize the fermentation process for the production of an alcohol such as butanol.

In some embodiments, co-products may be derived from the mash used in the fermentation process. For example, if corn is utilized as feedstock, corn oil can be separated from the mash and this corn oil contains triglycerides, fatty acids, diglycerides, monoglycerides, phospholipids, and antioxidants such as tocopherols. In some embodiments, corn oil may be used as a component of animal feed because its high triglyceride content is a source of metabolizable energy. In addition, the natural antioxidants in corn oil provide a source of vitamin E as well as reduce the development of rancidity.

Corn oil may optionally be added to other co-products at different rates and thus, for example, create the ability to vary the amount of triglyceride in the resulting co-product. In this manner, the fat content of the resulting co-product could be controlled, for example, to yield a lower fat, high protein animal feed that would better suit the needs of dairy cows compared to a high fat product.

In some embodiments, crude corn oil separated from mash may be further processed into edible oil for the food industry or direct use by consumers. For example, crude corn oil may be further processed to produce refined corn oil by degumming to remove phosphatides, alkali refining to neutralize free fatty acids, decolorizing for removal of color bodies and trace elements, winterizing to remove waxes, and deodorization (see, *e.g.*, Corn Oil, 5th Edition, Corn Refiners Association, Washington, D.C., 2006). The refined corn oil may be used, for example, by food manufacturers for the production of food products. The free fatty acids removed by alkali refining may be used as soapstock and waxes recovered from the winterizing step may be utilized in animal feeds.

In some embodiments, plant oils such as corn oil may be used as a feedstock for the generation of extractant for extractive fermentation. For example, oil derived from biomass may be converted into an extractant available for removal of a product alcohol such as butanol from a fermentation broth. The glycerides in the oil may be chemically or enzymatically converted into a reaction product, such as fatty acids, fatty alcohols, fatty amides, fatty acid alkyl esters, fatty acid glycol esters, and hydroxylated triglycerides, or mixtures thereof, which may be used a fermentation product extractant. Using corn oil as an example, corn oil triglycerides may be reacted with a base such as ammonia hydroxide or sodium hydroxide to obtain fatty amides, fatty acids, and glycerol. These fatty amides, fatty acids, or mixtures thereof may be used an extractant. In some embodiments, plant oil such as corn oil may be hydrolyzed by an enzyme such as lipase to form fatty acids (e.g., corn oil fatty acids). Methods for deriving extractants from biomass are described in U.S. Patent Application Publication No. 2011/0312044 and PCT International Publication No. WO 2011/159998, which are herein incorporated by reference.

In some embodiments, corn oil may also be used in the manufacture of resins, plastics, polymers, and lubricants, and may also be utilized by the pharmaceutical industry as a component of drug formulations. Corn oil may also be used in the manufacture of products such as printing inks, paint and varnish, soap, and textiles.

In some embodiments, corn oil can also be used as feedstock for biodiesel or renewable diesel. In some embodiments, plant oils or a combination of plant oils can also be used as feedstock for biodiesel or renewable diesel. Plant oils include, for example, canola, castor, corn, jojoba, karanja, mahua, linseed, soybean, palm, peanut, rapeseed, rice, safflower, and sunflower oils. Biodiesel may be derived from either the transesterification or esterification of plant oils with alcohols such as methanol, ethanol, and butanol. For example, biodiesel may be produced by acid-catalyzed, alkali-catalyzed, or enzyme-catalyzed transesterification or esterification (e.g., transesterification of plant oil-derived triglycerides or esterification of plant oil-derived free fatty acids). Inorganic acids such as sulfuric acid, hydrochloric acid, and phosphoric acid, organic acids such as toluenesulfonic acid and naphthalenesulfonic acid, or solid acids such as Amberlyst® sulfonated polystyrene resins, or zeolites may be used as a catalyst for acid-catalyzed transesterification or esterification. Bases such as potassium hydroxide, potassium methoxide, sodium hydroxide, sodium methoxide, or calcium hydroxide may be used as a catalyst for alkali-catalyzed transesterification or esterification. In some embodiments, biodiesel may be produced by an integrated process, for example, acid-catalyzed esterification of free fatty acids followed by base-catalyzed transesterification of triglycerides.

Enzymes such as lipases or esterases may be used to catalyze transesterification or esterification reactions. Lipases may be derived from bacteria or fungi, for example, *Pseudomonas, Thermomyces, Burkholderia, Candida,* and *Rhizomucor.* In some embodiments, lipases may be derived *Pseudomonas fluorescens, Pseudomonas cepacia, Rhizomucor miehei, Burkholderia cepacia, Thermomyces lanuginosa,* or *Candida antarctica*. In some embodiments, the enzyme may be immobilized on a soluble or insoluble support. The immobilization of enzymes may be performed using a variety of techniques including 1) binding of the enzyme to a porous or non-porous carrier support, via covalent support, physical adsorption, electrostatic binding, or affinity binding; 2) crosslinking with bifunctional or multifunctional reagents; 3) entrapment in gel matrices, polymers, emulsions, or some form of membrane; and 4) a combination of any of these methods. In some embodiments, lipase may be immobilized on, for example, acrylic resin, silica, or beads (e.g., polymethacrylate beads). In some embodiments, the lipases may be soluble.

Reactor configurations for the production of biodiesel include, for example, batch-stirred tank reactors, continuous-stirred tank reactors, packed bed reactors, fluid bed reactors, expanding bed reactors, and recirculation membrane reactors.

In some embodiments, biodiesel described herein may comprise one or more of the following fatty acid alkyl esters (FAAE): fatty acid methyl esters (FAME), fatty acid ethyl esters (FAEE), and fatty acid butyl esters (FABE). In some embodiments, biodiesel described herein may comprise one or more of the following: myristate, palmitate, stearate, oleate, linoleate, linolenate, arachidate, and behenate.

In some embodiments, extractant by-product can be used, all or in part, as a component of an animal feed by-product or it can be used as feedstock for biodiesel or renewable diesel. In some embodiments, oil from the fermentation process can be recovered by evaporation. This non-aqueous composition can comprise fatty acid esters (e.g., fatty acid isobutyl esters) and fatty acids and this composition (or stream) can be fed to a hydrolyser to recover isobutanol and fatty acids. In a further embodiment, this stream can be used as feedstock for biodiesel production.

In some embodiments, the biodiesel described herein meets the specifications of the American Society for Testing and Materials (ASTM) D6751. In some embodiments, the biodiesel described herein meets the specifications of the European standard EN 14214.

In some embodiments, a composition may comprise at least 2% biodiesel, at least 5% biodiesel, at least 10% biodiesel, at least 20% biodiesel, at least 30% biodiesel, at least 40% biodiesel, at least 50% biodiesel, at least 60% biodiesel, at least 70% biodiesel, at least 80% biodiesel, at least 90% biodiesel, or 100% biodiesel.

In some embodiments, the biodiesel described herein may be blended with a petroleum-based diesel fuel to form a biodiesel blend. In some embodiments, a biodiesel blend may comprise at least 2% by volume biodiesel, at least 3% by volume biodiesel, at least 4% by volume biodiesel, at least 5% by volume biodiesel, at least 6% by volume biodiesel, at least 7% by volume biodiesel, at least 8% by volume biodiesel, at least 9% by volume biodiesel, at least 10% by volume biodiesel, at least 11% by volume biodiesel, at least 12% by volume biodiesel, at least 13% by volume biodiesel, at least 14% by volume biodiesel, at least 15% by volume biodiesel, at least 16% by volume biodiesel, at least 17% by volume biodiesel, at least 18% by volume biodiesel, at least 19% by volume biodiesel, or at least 20% by volume biodiesel. In some embodiments, a biodiesel blend may comprise up to about 20% by volume biodiesel.

A by-product of biodiesel production is glycerol. In addition, glycerol may also be a by-product of the generation of extractant from plant oils and the fermentation process. A feedstock for biodiesel may be produce by reacting a COFA containing stream with glycerol. The reaction may be catalyzed by strong inorganic acids such as sulfuric acid or by solid acid catalysts such as Amberlyst™ polymeric catalysts and ion exchange resins. High conversions may be obtained by withdrawing water from the reaction mass. The reaction product will contain mono-, di-, and triglycerides in a proportion determine by the ratio of reactants and the extent of reaction. The glyceride mix may be used in lieu of the triglyceride feed normally used to make biodiesel. In some embodiments, the glycerides may be used as a surfactant or as a feedstock for biodiesel.

In some embodiments, solids can be separated from mash and can comprise triglycerides and free fatty acids. These solids (or stream) can be used as an animal feed, either recovered as discharge from centrifugation or after drying. The solids (or wet cake) can be particularly suited as feed for ruminants (e.g., dairy cows) because of its high content of available lysine and by-pass or rumen undegradable protein. For example, these solids can be of particular value in a high protein, low fat feed. In some embodiments, these solids can be used as a base, that is, other by-products such as syrup can be added to the solids to form a product that can be used as an animal feed. In some embodiments, different amounts of other by-products can be added to the solids to tailor the properties of the resulting product to meet the needs of a certain animal species.

The composition of solids separated from whole stillage can include, for example, crude protein, fatty acid, and fatty acid esters. In some embodiments, this composition (or by-product) can be used, wet or dry, as an animal feed where, for example, a high protein (e.g., high lysine), low fat, and high fiber content is desired. In some embodiments, fat can be added to this composition, for example, from another by-product stream if a higher fat, low fiber animal feed is desired. In some embodiments, this higher fat, low fiber animal feed can be used for swine or poultry. In a further embodiment, a non-aqueous composition of CDS can include, for example, protein, fatty acids, and fatty acid esters (e.g., fatty acid isobutyl esters) as well as other dissolved and suspended solids such as salts and carbohydrates. This CDS composition can be used, for example, as animal feed, either wet or dry, where protein, low fat, high mineral salt feed component is desired. In some embodiments, this composition can be used as a component of a dairy animal feed. In some embodiments, WDG may comprise protein, fiber, fat, and up to about 70% moisture. In some embodiments, DDGS may comprise about 10-12% moisture.

The various streams generated by the production of an alcohol (e.g., butanol) via a fermentation process can be combined in many ways to generate a number of co-products. For example, if crude corn from mash is used to generate fatty acids to be utilized as extractant and lipid is extracted by evaporators for other purposes, then the remaining streams can be combined and processed to create a co-product comprising crude protein, crude fat, triglycerides, fatty acids, and fatty acid esters such as fatty acid isobutyl esters (distillers co-products).

In some embodiments of the invention, the crude protein content of the distillers co-products (e.g., distillers co-products for animal feed) can be at least about 20% (percentage weight of the distillers co-products), at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95%. In some embodiments, the crude protein content is any range of values disclosed herein, for example, from about 20% to about 95%, from about 25% to about 95%, from about 30% to about 95%, from about 40% to about 95%, from about 50% to about 95%, from about 20% to about 80%, from about 30% to about 80%, from about 40% to about 80%, from about 50% to about 80%, from about 20% to about 50%, from about 30% to about 50%, from about 20% to about 45%, from about 25% to about 45%, from about 30% to about 45%, from about 20% to about 40%, or from about 25% to about 40%, from about 30% to about 40%, from about 20% to about 35%, from about 25% to about 35%, or from about 30% to about 35%.

In some embodiments of the invention, the crude fat content of the distillers co-products (e.g., distillers co-products for animal feed) can be less than about 10% (percentage weight of the distillers co-products), less than about 9%, less than about 8%, less than about 7%, less than about 6%, less than about 5%, less than about 4%, less than about 3%, less than about 2%, or less than about 1%. In some embodiments, the crude fat content is any range of values disclosed herein, for example, from about 1% to about 10%, from about 2% to about 10%, from about 3% to about 10%, from about 4% to about 10%, from about 5% to about 10%, from about 1% to about 8%, from about 2% to about 8%, from about 3% to about 8%, from about 4% to about 8%, from about 1% to about 5%, or from about 5% to about 10%.

In some embodiments of the invention, the fatty acid content of the distillers co-products can be less than about 10% (percentage weight of the distillers co-products), less than about 9%, less than about 8%, less than about 7%, less than about 6%, less than about 5%, less than about 4%, less than about 3%, less than about 2%, or less than about 1%. In some embodiments, the fatty acid content is any range of values disclosed herein, for example, from about 1% to about 10%, from about 2% to about 10%, from about 3% to about 10%, from about 4% to about 10%, from about 5% to about 10%, from about 1% to about 8%, from about 2% to about 8%, from about 3% to about 8%, from about 4% to about 8%, from about 1% to about 5%, or from about 5% to about 10%.

In some embodiments of the invention, the triglyceride content of the distillers co-products can be less than about 10%, less than about 9%, less than about 8%, less than about 7%, less than about 6%, less than about 5%, less than about 4%, less than about 3%, less than about 2%, or less than about 1%. In some embodiments, the triglyceride content is any range of values disclosed herein, for example, from about 1% to about 10%, from about 2% to about 10%, from about 3% to about 10%, from about 4% to about 10%, from about 5% to about 10%, from about 1% to about 8%, from about 2% to about 8%, from about 3% to about 8%, from about 4% to about 8%, from about 1% to about 5%, or from about 5% to about 10%.

In some embodiments of the invention, the lysine content of the distillers co-products can be less than about 10% (percentage weight of the distillers co-products), less than about 9%, less than about 8%, less than about 7%, less than about 6%, less than about 5%, less than about 4%, less than about 3%, less than about 2%, or less than about 1%. In some embodiments, the lysine content is any range of values disclosed herein, for example, from about 1% to about 10%, from about 2% to about 10%, from about 3% to about 10%, from about 4% to about 10%, from about 5% to about 10%, from about 1% to about 8%, from about 2% to about 8%, from about 3% to about 8%, from about 4% to about 8%, from about 1% to about 5%, or from about 5% to about 10%.

In some embodiments of the invention, the fatty acid ester content of the distillers co-products can be less than about 10% (percentage weight of the distillers co-products), less than about 9%, less than about 8%, less than about 7%, less than about 6%, less than about 5%, less than about 4%, less than about 3%, less than about 2%, or less than about 1%. In some embodiments, the fatty acid ester (e.g., fatty acid isobutyl ester) content is any range of values disclosed herein, for example, from about 1% to about 10%, from about 2% to about 10%, from about 3% to about 10%, from about 4% to about 10%, from about 5% to about 10%, from about 1% to about 8%, from about 2% to about 8%, from about 3% to about 8%, from about 4% to about 8%, from about 1% to about 5%, or from about 5% to about 10%.

In some embodiments, the distillers co-products (e.g., distillers co-products for animal feed) comprises at least about 20% to about 35% crude protein (percentage weight of the distillers co-products), at least about 1% to about 20% crude fat, at least about 0% to about 5% triglycerides, at least about 4% to about 10% fatty aci, and at least about 2% to about 6% fatty acid este (e.g., fatty acid isobutyl este). In some embodiments, the distiller co-products comprises about 25% crude protein, about 10% crude fat, about 0.5 % triglycerides, about 6% fatty acid, and about 4% fatty acid isobutyl ester.

In some embodiments, the distillers co-products (e.g., distillers co-products for animal feed) may comprise one or more of the following: protein, fat, fiber, vitamins, and minerals. In some embodiments, the distillers co-products may be supplemented with amino acids, vitamins, and minerals. For example, distillers co-products may be supplemented with amino acids such as histidine, isoleucine, leucine, lysine, methionine, phenylalanine, threonine, tryptophan, and valine as well as other amine acids such as alanine, arginine, aspartic acid, asparagine, cysteine, glutamic acid, glutamine, glycine, hydroxylysine, hydroxyproline, lanthionine, ornithine, proline, serine, and tyrosine. Distillers co-products may be supplemented with minerals such as calcium, chloride, cobalt, copper, fluoride, iodine, iron, magnesium, manganese, phosphorus, potassium, selenium, sodium, sulfur, and zinc. Distillers co-products may be supplemented with vitamins such as vitamins A, C, D, E, K, and B (thiamine, riboflavin, niacin, pantothenic acid, biotin, vitamin B6, vitamin B12 and folate). In some embodiments, the distillers co-products may comprise triglycerides, fatty acids, fatty acid esters, and glycerol.

In some embodiments, the moisture, protein, fat, fiber, and ash content of distillers co-products may be measured (see, e.g., www.aoac.org, www.foragetesting.org, www.aocs.org). In some embodiments, the moisture content of distillers co-products may be measured using analytical methods such as Association of Analytical Communities (AOAC) 934.01, AOAC 935.29, AOAC 930.15, AOAC 2001.12, and National Forgaing Testing Association (NFTA) 2.2.2.5. In some embodiments, the protein content of distillers co-products may be measured using analytical methods such as AOAC 990.03 and AOAC 2001.11. In some embodiments, the fat content of distillers co-products may be measured using analytical methods such as AOAC 2003.5, AOAC 2003.06, AOAC 920.39, AOAC 954.02 and AOAC 945.16. In some embodiments, the fiber content of distillers co-products may be measured using analytical methods such as AOAC 978.10, AOAC 962.09, and American Oil Chemists' Society (AOCS) Ba 6a-05. In some embodiments, the ash content of distillers co-products may be measured using analytical methods such as AOAC 942.05.

Some embodiments of the present invention are directed to methods of producing a high value animal feed component from an ethanol or butanol production process, comprising providing an ethanol or butanol production process with at least one process feedstream to optimize crude protein and crude fat content for an animal feed market. In some embodiments, at least two process feedstreams are combined to optimize crude protein and crude fat content for an animal feed or animal feed market. In some embodiments, at least three process feedstreams are combined to optimize crude protein and crude fat content for an animal feed or animal feed market.

In some embodiments, a distillers co-products for animal feed composition or method of producing a distillers co-products for animal feed has been optimized to the type of available feedstream. In some embodiments, a distillers co-products for animal feed for the cattle feed market of method of producing a distillers co-products for animal feed for the cattle feed market comprises wet process feedstreams, e.g., WDGS or WDG.

In some embodiments, an animal feed market related to the methods and compositions of the present invention is a livestock feed market, ruminant feed market, cattle feed market, dairy animal feed market, swine feed market, goat feed market, sheep feed market, poultry feed market, equine feed market, aquaculture feed market, domestic pet feed market, or any combination thereof. In some embodiments, an animal feed related to the methods and compositions of the present invention is a livestock feed, ruminant feed, cattle feed, dairy animal feed (e.g., dairy cow feed), swine feed, goat feed, sheep feed, poultry feed, equine feed, aquaculture feed, domestic pet feed, or any combination thereof.

In some embodiments, a distillers co-products comprises the fat and protein content described in Table 6 (e.g., DCP1, DCP2, or DCP3), or a substantially similar fat and protein content described in Table 6. In some embodiments, a distillers co-products comprises the fat, protein and lipid content described in Table 6 (e.g., DCP1, DCP2, or DCP3), or a substantially similar fat and protein content described in Table 6. In some embodiments, a distillers co-products comprises the fat, protein, lipid and lysine content described in Table 6 (*e.g.*, DCP1, DCP2, or DCP3), or a substantially similar fat and protein content described in Table 6.

In some embodiments, a distillers co-products comprising the fat and protein content of DCP1 in Table 6, or a substantially similar fat and protein content of DCP1 in Table 6, has a nutrient profile for cattle feed, dairy animal feed (e.g., dairy cow feed), or swine feed, or a cattle feed market, dairy animal feed market (e.g., dairy animal feed market), or a swine feed market. In some embodiments, a distillers co-products comprising the fat, protein, and lipid content of DCP1 in Table 6, or a substantially similar fat, protein, and lipid content of DCP1 in Table 6, has a nutrient profile for cattle feed, dairy animal feed (e.g., dairy cow feed), or swine feed, or a cattle feed market, dairy animal feed market (e.g., dairy animal feed market), or a swine feed market. In some embodiments, a distillers co-products comprising the fat, protein, lipid, and lysine content of DCP1 in Table 6, or a substantially similar fat, protein, lipid, and lysine content of DCP1 in Table 6, has a nutrient profile for cattle feed, dairy animal feed (e.g., dairy cow feed), or swine feed, or a cattle feed market, dairy animal feed market (e.g., dairy animal feed market), or a swine feed market.

In some embodiments, a distillers co-products comprising the fat and protein content of DCP2 in Table 6, or a substantially similar fat and protein content of DCP2 in Table 6, has a nutrient profile for cattle feed or dairy animal feed (e.g., dairy cow feed), or a cattle feed market or dairy animal feed market (e.g., dairy animal feed market). In some embodiments, a distillers co-products comprising the fat, protein, and lipid content of DCP2 in Table 6, or a substantially similar fat, protein, and lipid content of DCP2 in Table 6, has a nutrient profile for cattle feed or dairy animal feed (e.g., dairy cow feed), or a cattle feed market or dairy animal feed market (e.g., dairy animal feed market. In some embodiments, a distillers co-products comprising the fat, protein, lipid, and lysine content of DCP2 in Table 6, or a substantially similar fat, protein, lipid and lysine content of DCP2 in Table 6, has a nutrient profile for cattle feed or dairy animal feed (e.g., dairy cow feed), or a cattle feed market or dairy animal feed market (e.g., dairy animal feed market).

In some embodiments, a distillers co-products comprising the fat and protein content of DCP3 in Table 6, or a substantially similar fat and protein content of DCP3 in Table 6, has a nutrient profile for cattle feed, dairy animal feed (e.g., dairy cow feed), swine feed, or poultry feed (e.g., chicken feed), or a cattle feed market, dairy animal feed market (e.g., dairy animal feed market), swine feed market, or poultry feed market (e.g., chicken feed market). In some embodiments, a distillers co-products comprising the fat, protein, and lipid content of DCP3 in Table 6, or a substantially similar fat, protein, and lipid content of DCP3 in Table 6, has a nutrient profile for cattle feed, dairy animal feed (e.g., dairy cow feed), swine feed, or poultry feed (e.g., chicken feed), or a cattle feed market, dairy animal feed market (e.g., dairy animal feed market), swine feed market, or poultry feed market (e.g., chicken feed market). In some embodiments, a distillers co-products comprising the fat, protein, lipid and lysine content of DCP3 in Table 6, or a substantially similar fat, protein, lipid and lysine content of DCP3 in Table 6, has a nutrient profile for cattle feed, dairy animal feed (e.g., dairy cow feed), swine feed, or poultry feed (e.g., chicken feed), or a cattle feed market, dairy animal feed market (e.g., dairy animal feed market), swine feed market, or poultry feed market (e.g., chicken feed market).

In some embodiments, the methods of the present invention further comprise supplementing a distillers co-products (e.g., distillers co-products for animal feed) composition with one or more additional components. In some embodiments, an additional component is a nutrient, flavor enhancer, digestion stimulant, or color enhancer. In some embodiments, the methods of the present invention further comprise the recycling of at least one feedstream into the ethanol or butanol production process.

In some embodiments, the protein content of the distillers co-products for animal feed is supplemented with a yeast cell mass, wherein the yeast cell mass increases the protein content of the distillers co-products for animal feed. In some embodiments, the fatty acid content provides additional energy and nutrient for an animal feed composition that comprises the distillers co-products for animal feed. In some embodiments, the butanol produced in the butanol production process is used as a solvent wash for at least one feedstream of the butanol production process. In some embodiments, the alcohol produced in the alcohol production process is used as a solvent wash for at least one feedstream of the ethanol production process. In some embodiments, a first feedstream is combined with a second feedstream to produce a distillers co-products for animal feed with increased storage stability. In some embodiments, the distillers co-products (e.g., distillers co-products for animal feed) has an improved color profile.

Some embodiments of the present invention relate to a distillers co-products for animal feed composition comprising at least about 25% crude protein (weight percentage of the composition). In some embodiments, the distillers co-products for animal feed composition further comprises less than about 10% crude fat. In some embodiments, a distiller co-products for animal feed composition comprises about 7% crude fat and at least 13% crude protein. In some embodiments, the distillers co-products for animal feed composition further comprises less than about 10% fatty acid ester (e.g., fatty acid isobutyl ester). In some embodiments, the distillers co-products for animal feed composition further comprises less than about 5% lysine. In some embodiments, such compositions have a nutrient profile for an animal feed or animal feed market.

In some embodiments, the distillers co-products for animal feed composition has high levels of fatty acids and has the nutrient profile for swine or poultry feed or for the swine or poultry feed market. In some embodiments, a distillers co-products for animal feed composition has high levels of fatty acids and fiber and has the nutrient profile.

In some embodiments, a process feedstream of solids removed from a mash before fermentation (e.g., wet cake) has a high protein and low fat content. In some embodiments, a distillers co-products for animal feed composition comprising such a feedstream has the nutrient profile for dairy animal feed or for the dairy animal feed market.

In some embodiments, the lipid is generated by evaporators and the fatty acids are used for other purposes and about 50% (weight percentage) of the crude corn from mash and the remaining streams are combined and processed, the resulting distillers co-products can comprise crude protein, crude fat, triglycerides, fatty acid, and fatty acid ester. In some embodiments, the distillers co-products comprises at least about 25% to about 31 % crude protein, at least about 6% to about 10% crude fat, at least about 4% to about 8% triglycerides, at least about 0% to about 2% fatty acid, and at least about 1% to about 3% fatty acid ester (e.g., fatty acid isobutyl ester). In some embodiments, the distillers co-products comprises from about 28% crude protein, about 8% crude fat, about 6% triglycerides, about 0.7% fatty acid, and about 1% fatty acid ester (e.g., ).

In some embodiments, the solids separated from whole stillage and about 50% of the corn oil extracted from mash are combined and the resulting distillers co-products composition can comprise crude protein, crude fat, triglycerides, fatty acid, fatty acid isobutyl ester, lysine, NDF, and ADF. In some embodiments, the distillers co-products comprises from at least about 26% to about 34% crude protein, at least about 15% to about 25% crude fat, at least about 12% to about 20% triglycerides, at least about 1% to about 2% fatty acid, at least about 2% to about 4% fatty acid ester (e.g., fatty acid isobutyl ester), at least about 1% to about 2% lysine, at least about 11% to about 23% NDF, and at least about 5% to about 11% ADF. In some embodiments, the distillers co-products can comprise about 29% crude protein, about 21% crude fat, about 16% triglycerides, about 1% fatty acid, about 3% fatty acid ester (e.g., fatty acid isobutyl ester), about 1% lysine, about 17% NDF, and about 8% ADF. The high fat, triglyceride, and lysine content and the lower fiber content of this distillers co-products can be desirable as feed for swine and poultry.

In some embodiments, distillers co-products such as DDGS may comprise one or more of the following: about 20-35% crude protein, about 5-15% crude fat, about 5-10% crude fiber, about 0-10% ash, about 0-2% lysine, about 0-2% arginine, about 0-0.5% tryptophan, about 0-1% methionine, and about 0-1% phosphorus.

In some embodiments, distillers co-products may be processed by densification or pelleting. For example, pelleting DDGS for animal feed may stimulate feed intake (i.e., improved palatability); improve nutrient and bulk density; and improve durability, handling, and flowability in feeding bins. Pelletized distillers co-products may also reduce transportation costs. Several factors such as the physical (e.g., particle size, density and nutrient (e.g., protein, fat, fiber, moisture, oil content) characteristics of the DDGS and the pellet mill operations (e.g., die specifications, die speed, die geometry, conditioning time and temperature) may have an impact on the pellet quality. In some embodiments, a method of pelletizing distillers co-products may include adjusting die specifications, die speed, die geometry, conditioning time, and conditioning temperature to improve the quality of distillers co-products pellets. In some embodiments, the pelletized distillers co-products may be spherical, cylindrical, or cube shaped.

In some embodiments, DDGS may be further processed to separate fiber producing DDGS with reduced fiber and increased fat and protein content. In some embodiments, fiber may be removed from DDGS by elutriation and/or sieving (see, e.g., Srinivasin, et al., Cereal Chem. 83:324-330, 2006). The fiber removed from DDGS may be used in feed for ruminant animals or to produce fiber oil, fiber gum,or xylitol. Fiber may also be used as an energy source.

In some embodiments, co-products of the present invention such as DDGS may be used for human consumption. For example, DDGS may be used as a flour supplement, for example in baked goods. DDGS may also be utilized in the agricultural industry as a supplement for soils, for example, as a fertilizer. In some embodiments, distillers grains may be used to produce biogas (e.g., methane, CO₂) via an anaerobic digester, and the biogas may be used as an energy source, for example, for the production of heat and electricity. In some embodiments, pelletized distillers co-products may be used as fuel. For example, pelletized DDGS may be mixed with coal forming a fuel blend that may be used as an energy source.

In some embodiments, a mash comprising fermentable sugars can be further converted to end products such as high fructose sugars. In other embodiments the fermentable sugars are subjected to fermentation with fermenting microorganisms. The contacting step and the fermenting step can be performed simultaneously in the same reaction vessel or sequentially. In general, fermentation processes are described in The Alcohol Textbook 3rd ED, A Reference for the Beverage, Fuel and Industrial Alcohol Industries, Eds Jacques et al., (1999) Nottingham University Press, UK.

In some embodiments, the butanol yield from the butanol production process related to the present invention is at least about 1 g/L, at least about 2 g/L, at least about 3 g/L, at least about 4 g/L, or at least about 5 g/L. In some embodiments, the butanol yield can be any range of values disclosed herein, for example, from about 1 g/L to about 5 g/L, from about 2 g/L to about 5 g/L, from about 3 g/L to about 5 g/L, from about 4 g/L to about 5 g/L, from about 1 g/L to about 4 g/L, from about 2 g/L to about 4 g/L, from about 3 g/L to about 4 g/L, from about 1 g/L to about 3 g/L, from about 2 g/L to about 3 g/L, or from about 1 g/L to about 2 g/L.

In some embodiments, the ethanol yield from the ethanol production process related to the present invention is at least about 1 g/L, at least about 2 g/L, at least about 3 g/L, at least about 4 g/L, or at least about 5 g/L. In some embodiments, the butanol yield can be any range of values disclosed herein, for example, from about 1 g/L to about 5 g/L, from about 2 g/L to about 5 g/L, from about 3 g/L to about 5 g/L, from about 4 g/L to about 5 g/L, from about 1 g/L to about 4 g/L, from about 2 g/L to about 4 g/L, from about 3 g/L to about 4 g/L, from about 1 g/L to about 3 g/L, from about 2 g/L to about 3 g/L, or from about 1 g/L to about 2 g/L.

Other embodiments of the present invention are directed to methods of mitigating the impact of fermentation contaminants on the production of a distillers co-products for animal feed, comprising separating at least one feedstream of an ethanol or butanol production process prior to fermentation. In some embodiments, the present invention is directed to methods of reducing lipid content variability of a distillers co-products for animal feed, comprising separating feedstreams of an ethanol or butanol production process contributing to a distillers co-products for animal feed production, and combining the feedstreams to achieve a controlled lipid content. In some embodiments, the present invention is directed to methods of increasing triglyceride content of a distillers co-products for animal feed, comprising combining higher triglyceride-containing feedstreams of an ethanol or butanol production process at an increasing ratio than lower triglyceride-containing streams for a particular distillers co-products for animal feed composition.

In some embodiments, the present invention is directed to methods of reducing mycotoxin contamination in a distillers co-products for animal feed. Mycotoxins such as aflatoxin, trichotecenes such as deoxynivalenol (vomitoxin) and T-2 toxin, fumonisin, zearalenone may be present in the distillers co-products. Distillers co-products may be analyzed for the presence of mycotoxins using high performance liquid chromatography (HPLC), thin layer chromatography, gas liquid chromatography (GLC), or enzyme-linked immunosorbent assay (ELISA) (see, e.g., Neogen Corporation, Lansing, MI). In some embodiments, the present invention is directed to methods of reducing mycotoxin contamination in a distillers co-products for animal feed comprising separating feedstreams of an alcohol production process contributing to distillers co-products for animal feed production, testing the feedstream for mycotoxins, and eliminating or purifying feedstreams with mycotoxin contamination potential. In some embodiments, the contaminated feedstream may be treated to eliminate the contamination. For example, mycotoxin contaminated grains may be treated by ammoniation. In some embodiments, mold inhibitors may be added to distillers co-products. For example, mold inhibitors such as ammonia; organic acids such as propionic acid, sorbic acid, benzoic acid, and acetic acid, and salts of organic acids such as calcium propionate and potassium sorbate may be added to distillers co-products. Adsorbent materials such as clays and activated charcoal may be added to distillers co-products to minimize mycotoxin contamination. Mycotoxin contamination may also be minimized by the presence of esters generated by the conversion of an alcohol to an ester. For example, the methods described herein generate alcohol esters (e.g., butyl esters) by esterification of a fatty acid with an alcohol. In some embodiments, distillers co-products may comprise esters such as butyl esters as a means to minimize mycotoxin contamination.

Yeast cells are generally supplied in amounts of from 10⁴ to 10¹² viable yeast count per ml of fermentation broth. In some embodiments, yeast cells are supplied in amounts of from 10⁷ to 10¹⁰ viable yeast count per ml of fermentation broth. The fermentation can include in addition to a fermenting microorganisms (*e.g*., yeast) nutrients, optionally acid and additional enzymes. In some embodiments, in addition to the raw materials described above, fermentation media will contain supplements including but not limited to vitamins (*e.g*., biotin, folic acid, nicotinic acid, riboflavin), cofactors, and macro and micro-nutrients and salts (*e.g*., (NH₄)₂SO₄; K₂HPO₄; NaCl; MgSO₄; H₃BO₃; ZnCl₂; and CaCl₂).

While not wishing to be bound by theory, it is believed that the processes described herein are useful in conjunction with any alcohol producing microorganism. Alcohol-producing microorganisms are known in the art. For example, fermentative oxidation of methane by methanotrophic bacteria (e.g., *Methylosinus trichosporium*) produces methanol, and contacting methanol (a C₁ alkyl alcohol) with a carboxylic acid and a catalyst capable of esterifying the carboxylic acid with methanol forms a methanol ester of the carboxylic acid. The yeast strain CEN.PK113-7D (CBS 8340, the Centraal Buro voor Schimmelculture; van Dijken, et al., Enzyme Microb. Techno. 26:706-714, 2000) can produce ethanol, and contacting ethanol with a carboxylic acid and a catalyst capable of esterifying the carboxylic acid with the ethanol forms ethyl ester.

Recombinant microorganisms which produce alcohol are also known in the art (e.g., Ohta, et al., Appl. Environ. Microbiol. 57:893-900, 1991; Underwood, et al., Appl. Environ. Microbiol. 68:1071-1081, 2002; Shen and Liao, Metab. Eng. 10:312-320, 2008; Hahnai, et al., Appl. Environ. Microbiol. 73:7814-7818, 2007; U.S. Patent No. 5,514,583; U.S. Patent No. 5,712,133; PCT International Publication No. WO 1995/028476; Feldmann, et al., Appl. Microbiol. Biotechnol. 38: 354-361, 1992; Zhang, et al., Science 267:240-243, 1995; U.S. Patent Application Publication No. 2007/0031918; U.S. Patent No. 7,223,575; U.S. Patent No. 7,741,119; U.S. Patent Application Publication No. 2009/0203099; U.S. Patent Application Publication No. 2009/0246846; and PCT International Publication No. WO 2010/075241).

As mentioned above, the metabolic pathways of microorganisms may be genetically modified to produce an alcohol (e.g., butanol). For example, the microorganism may be engineered to contain a butanol biosynthetic pathway or a biosynthetic pathway for a butanol isomer such as 1-butanol, 2-butanol, or isobutanol.

In some embodiments, the biosynthetic pathway comprises at least one heterologous polynucleotide encoding a polypeptide which catalyzes a substrate to product conversion of the biosynthetic pathway. In some embodiments, each substrate to product conversion of the biosynthetic pathway is catalyzed by a polypeptide encoded by a heterologous polynucleotide. These biosynthetic pathways may also be modified to reduce or eliminate undesired metabolites, and thereby improve yield of the alcohol.

The production of butanol by a microorganism is disclosed, for example, in U.S. Patent Application Publication Nos. 2007/0092957; 2007/0259410; 2007/0292927; 2008/0182308; 2008/0274525; 2009/0305363; and 2009/0305370.

Suitable recombinant microorganisms capable of producing butanol are known in the art, and certain suitable microorganisms capable of producing butanol are described herein. Recombinant microorganisms to produce butanol via a biosynthetic pathway can include a member of the genera *Clostridium, Zymomonas, Escherichia, Salmonella, Serratia, Erwinia, Klebsiella, Shigella, Rhodococcus, Pseudomonas, Bacillus, Lactobacillus, Enterococcus, Alcaligenes, Klebsiella, Paenibacillus*, *Arthrobacter, Corynebacterium, Brevibacterium, Schizosaccharomyces, Kluyveromyces, Yarrowia, Pichia, Zygosaccharomyces, Debaryomyces, Candida, Brettanomyces, Pachysolen, Hansenula, Issatchenkia, Trichosporon, Yamadazyma,* or *Saccharomyces.* In some embodiments, recombinant microorganisms can be selected from the group consisting of *Escherichia coli, Alcaligenes eutrophus, Bacillus lichenifonnis, Paenibacillus macerans, Rhodococcus erythropolis, Pseudomonas putida, Lactobacillus plantarum, Enterococcus faecium, Enterococcus gallinarium, Enterococcus faecalis, Bacillus subtilis, Candida sonorensis, Candida methanosorbosa, Kluyveromyces lactis, Kluyveromyces marxianus, Kluyveromyces thermotolerans, Issatchenkia orientalis, Debaryomyces hansenii, and Saccharomyces cerevisiae.* In some embodiments, the recombinant microorganism is yeast. In some embodiments, the recombinant microorganism is crabtree-positive yeast selected from *Saccharomyces, Zygosaccharomyces, Schizosaccharomyces, Dekkera, Torulopsis, Brettanomyces,* and some species of *Candida.* Species of crabtree-positive yeast include, but are not limited to, *Saccharomyces cerevisiae, Saccharomyces kluyveri, Schizosaccharomyces pombe, Saccharomyces bayanus, Saccharomyces mikitae, Saccharomyces paradoxus, Saccharomyces uvarum, Saccharomyces castelli, Saccharomyces kluyveri, Zygosaccharomyces rouxii, Zygosaccharomyces bailli,* and *Candida glabrata.* Suitable strains include those described in certain applications cited and incorporated by reference herein as well as in U.S. Provisional Application No. 61/380,563, filed on September 7, 2010 and PCT International Publication No. WO 2012/033832. Suitable strains and methods also include those described in U.S. Provisional Application No. 61/246,709, filed on September 29,2010.

Examples of other fermenting organisms such as those that produce ethanol, for example, ethanologenic bacteria which express alcohol dehydrogenase and pyruvate dehydrogenase and which can be obtained from *Zymomonas moblis* (*see, e.g.,* U.S. Patent No. 5,000,000; U.S. Patent No. 5,028,539, U.S. Patent No. 5,424,202; U.S. Patent No. 5,514,583 and U.S. Patent No. 5,554,520) can be modified for butanol production. In additional embodiments, the isobutanologens express xylose reductase and xylitol dehydrogenase, enzymes that convert xylose to xylulose. In some embodiments, xylose isomerase is used to convert xylose to xylulose. In some embodiments, a microorganism capable of fermenting both pentoses and hexoses to butanol are utilized.

In some embodiments, microorganisms comprise a butanol biosynthetic pathway. In some embodiments, at least one, at least two, at least three, or at least four polypeptides catalyzing substrate to product conversions of a pathway are encoded by heterologous polynucleotides in the microorganism. In some embodiments, all polypeptides catalyzing substrate to product conversions of a pathway are encoded by heterologous polynucleotides in the microorganism. In some embodiments, the microorganism comprises a reduction or elimination of pyruvate decarboxylase activity. Microorganisms substantially free of pyruvate decarboxylase activity are described in US Application Publication No. 2009/0305363. Microorganisms substantially free of an enzyme having NAD-dependent glycerol-3-phosphate dehydrogenase activity such as GPD2 are also described therein.

Suitable biosynthetic pathways for production of butanol are known in the art, and certain suitable pathways are described herein. In some embodiments, the butanol biosynthetic pathway comprises at least one gene that is heterologous to the host cell. In some embodiments, the butanol biosynthetic pathway comprises more than one gene that is heterologous to the host cell. In some embodiments, the butanol biosynthetic pathway comprises heterologous genes encoding polypeptides corresponding to every step of a biosynthetic pathway.

Biosynthetic pathways for the production of isobutanol that may be used include those described in U.S. Patent No. 7,851,188. In one embodiment, the isobutanol biosynthetic pathway comprises the following substrate to product conversions:
a) pyruvate to acetolactate, which may be catalyzed, for example, by acetolactate synthase;
b) acetolactate to 2,3-dihydroxyisovalerate, which may be catalyzed, for example, by acetohydroxy acid reductoisomerase;
c) 2,3-dihydroxyisovalerate to α-ketoisovalerate, which may be catalyzed, for example, by acetohydroxy acid dehydratase;
d) α-ketoisovalerate to isobutyraldehyde, which may be catalyzed, for example, by a branched-chain α-keto acid decarboxylase; and,
e) isobutyraldehyde to isobutanol, which may be catalyzed, for example, by a branched-chain alcohol dehydrogenase.

In another embodiment, the isobutanol biosynthetic pathway comprises the following substrate to product conversions:
a) pyruvate to acetolactate, which may be catalyzed, for example, by acetolactate synthase;
b) acetolactate to 2,3-dihydroxyisovalerate, which may be catalyzed, for example, by ketol-acid reductoisomerase;
c) 2,3-dihydroxyisovalerate to α-ketoisovalerate, which may be catalyzed, for example, by dihydroxyacid dehydratase;
d) α-ketoisovalerate to valine, which may be catalyzed, for example, by transaminase or valine dehydrogenase;
e) valine to isobutylamine, which may be catalyzed, for example, by valine decarboxylase;
f) isobutylamine to isobutyraldehyde, which may be catalyzed by, for example, omega transaminase; and,
g) isobutyraldehyde to isobutanol, which may be catalyzed, for example, by a branched-chain alcohol dehydrogenase.

In another embodiment, the isobutanol biosynthetic pathway comprises the following substrate to product conversions:
a) pyruvate to acetolactate, which may be catalyzed, for example, by acetolactate synthase;
b) acetolactate to 2,3-dihydroxyisovalerate, which may be catalyzed, for example, by acetohydroxy acid reductoisomerase;
c) 2,3-dihydroxyisovalerate to α-ketoisovalerate, which may be catalyzed, for example, by acetohydroxy acid dehydratase;
d) α-ketoisovalerate to isobutyryl-CoA, which may be catalyzed, for example, by branched-chain keto acid dehydrogenase;
e) isobutyryl-CoA to isobutyraldehyde, which may be catalyzed, for example, by acelylating aldehyde dehydrogenase; and,
f)isobutyraldehyde to isobutanol, which may be catalyzed, for example, by a branched-chain alcohol dehydrogenase.

Biosynthetic pathways for the production of 1-butanol that may be used include those described in U.S. Patent Application Publication No. 2008/0182308. In one embodiment, the 1-butanol biosynthetic pathway comprises the following substrate to product conversions:
a) acetyl-CoA to acetoacetyl-CoA, which may be catalyzed, for example, by acetyl-CoA acetyltransferase;
b) acetoacetyl-CoA to 3-hydroxybutyryl-CoA, which may be catalyzed, for example, by 3-hydroxybutyryl-CoA dehydrogenase;
c) 3-hydroxybutyryl-CoA to crotonyl-CoA, which may be catalyzed, for example, by crotonase;
d) crotonyl-CoA to butyryl-CoA, which may be catalyzed, for example, by butyryl-CoA dehydrogenase;
e) butyryl-CoA to butyraldehyde, which may be catalyzed, for example, by butyraldehyde dehydrogenase; and,
f) butyraldehyde to 1-butanol, which may be catalyzed, for example, by butanol dehydrogenase.

Biosynthetic pathways for the production of 2-butanol that may be used include those described in U.S. Patent Application Publication No. 2007/0259410 and U.S. Patent Application Publication No. 2009/0155870. In one embodiment, the 2-butanol biosynthetic pathway comprises the following substrate to product conversions:
a) pyruvate to alpha-acetolactate, which may be catalyzed, for example, by acetolactate synthase;
b) alpha-acetolactate to acetoin, which may be catalyzed, for example, by acetolactate decarboxylase;
c) acetoin to 3-amino-2-butanol, which may be catalyzed, for example, acetonin aminase;
d) 3-amino-2-butanol to 3-amino-2-butanol phosphate, which may be catalyzed, for example, by aminobutanol kinase;
e) 3-amino-2-butanol phosphate to 2-butanone, which may be catalyzed, for example, by aminobutanol phosphate phosphorylase; and,
f) 2-butanone to 2-butanol, which may be catalyzed, for example, by butanol dehydrogenase.

In another embodiment, the 2-butanol biosynthetic pathway comprises the following substrate to product conversions:
a) pyruvate to alpha-acetolactate, which may be catalyzed, for example, by acetolactate synthase;
b) alpha-acetolactate to acetoin, which may be catalyzed, for example, by acetolactate decarboxylase;
c) acetoin to 2,3-butanediol, which may be catalyzed, for example, by butanediol dehydrogenase;
d) 2,3-butanediol to 2-butanone, which may be catalyzed, for example, by dial dehydratase; and,
e) 2-butanone to 2-butanol, which may be catalyzed, for example, by butanol dehydrogenase.

Biosynthetic pathways for the production of 2-butanone that may be used include those described in U.S. Patent Application Publication No. 2007/0259410 and U.S. Patent Application Publication No. 2009/0155870. In one embodiment, the 2-butanone biosynthetic pathway comprises the following substrate to product conversions:
a) pyruvate to alpha-acetolactate, which may be catalyzed, for example, by acetolactate synthase;
b) alpha-acetolactate to acetoin, which may be catalyzed, for example, by acetolactate decarboxylase;
c) acetoin to 3-amino-2-butanol, which may be catalyzed, for example, acetonin aminase;
d) 3-amino-2-butanol to 3-amino-2-butanol phosphate, which may be catalyzed, for example, by aminobutanol kinase; and,
e) 3-amino-2-butanol phosphate to 2-butanone, which may be catalyzed, for example, by aminobutanol phosphate phosphorylase.

In another embodiment, the 2-butanone biosynthetic pathway comprises the following substrate to product conversions:
a) pyruvate to alpha-acetolactate, which may be catalyzed, for example, by acetolactate synthase;
b) alpha-acetolactate to acetoin which may be catalyzed, for example, by acetolactate decarboxylase;
c) acetoin to 2,3-butanediol, which may be catalyzed, for example, by butanediol dehydrogenase;
d) 2,3-butanediol to 2-butanone, which may be catalyzed, for example, by diol dehydratase.

In one embodiment, the invention produces butanol from plant-derived carbon sources, avoiding the negative environmental impact associated with standard petrochemical processes for butanol production. In one embodiment, the invention provides a method for the production of butanol using recombinant industrial host cells comprising a butanol pathway.

In some embodiments, the isobutanol biosynthetic pathway comprises at least one polynucleotide, at least two polynucleotides, at least three polynucleotides, or at least four polynucleotides that is/are heterologous to the host cell. In embodiments, each substrate to product conversion of an isobutanol biosynthetic pathway in a recombinant host cell is catalyzed by a heterologous polypeptide. In embodiments, the polypeptide catalyzing the substrate to product conversions of acetolactate to 2,3-dihydroxyisovalerate and/or the polypeptide catalyzing the substrate to product conversion of isobutyraldehyde to isobutanol are capable of utilizing NADH as a cofactor.

The terms "acetohydroxyacid synthase," "acetolactate synthase," and "acetolactate synthetase" (abbreviated "ALS") are used interchangeably herein to refer to an enzyme that catalyzes the conversion of pyruvate to acetolactate and CO₂. Example acetolactate synthases are known by the EC number 2.2.1.6 (Enzyme Nomenclature 1992, Academic Press, San Diego). These unmodified enzymes are available from a number of sources, including, but not limited to, *Bacillus subtilis* (GenBank Nos: CAB15618, Z99122), NCBI (National Center for Biotechnology Information) amino acid sequence, NCBI nucleotide sequence, respectively), *Klebsiella pneumoniae* (GenBank Nos: AAA25079), M73842), and *Lactococcus lactis* (GenBank Nos: AAA25161, L16975).

The term "ketol-acid reductoisomerase" ("KARI"), "acetohydroxy acid isomeroreductase," and "acetohydroxy acid reductoisomerase" will be used interchangeably and refer to enzymes capable of catalyzing the reaction of (S)-acetolactate to 2,3-dihydroxyisovalerate. Example KARI enzymes may be classified as EC number EC 1.1.1.86 (Enzyme Nomenclature 1992, Academic Press, San Diego), and are available from a vast array of microorganisms, including, but not limited to, *Escherichia coli* (GenBank Nos: NP_418222, NC_000913), *Saccharomyces cerevisiae* (GenBank Nos: NP_013459, NC_001144), *Methanococcus maripaludis* (GenBank Nos: CAF30210, BX957220), and *Bacillus subtilis* (GenBank Nos: CAB14789, Z99118). KARIs include *Anaerostipes caccae* KARI variants "K9G9" and "K9D3." Ketol-acid reductoisomerase (KARI) enzymes are described in U.S. Patent Application Publication Nos. 2008/0261230, 2009/0163376, and 2010/0197519, and PCT Application Publication No. WO/2011/041415. Examples of KARIs disclosed therein are those from *Lactococcus lactis, Vibrio cholera, Pseudomonas aeruginosa* PAO1, and *Pseudomonas fluorescens* PF5 mutants In some embodiments, the KARI utilizes NADH. In some embodiments, the KARI utilizes NADPH.

The term "acetohydroxy acid dehydratase" and "dihydroxyacid dehydratase" ("DHAD") refers to an enzyme that catalyzes the conversion of 2,3-dihydroxyisovalerate to α-ketoisovalerate. Example acetohydroxy acid dehydratases are known by the EC number 4.2.1.9. Such enzymes are available from a vast array of microorganisms, including, but not limited to, *E. coli* (GenBank Nos: YP_026248, NC000913), *Saccharomyces cerevisiae* (GenBank Nos: NP_012550, NC 001142), *M. maripaludis* (GenBank Nos: CAF29874 BX957219), *B. subtilis* (GenBank Nos: CAB14105, Z99115), *L. lactis,* and *N. crassa.* U.S. Patent Application Publication No. 2010/0081154, and U.S. Patent No. 7,851,188, describe dihydroxyacid dehydratases (DHADs), including a DHAD from *Streptococcus mutans.*

The term "branched-chain α-keto acid decarboxylase," "α-ketoacid decarboxylase," "α-ketoisovalerate decarboxylase," or "2-ketoisovalerate decarboxylase" ("KIVD") refers to an enzyme that catalyzes the conversion of α-ketoisovalerate to isobutyraldehyde and CO₂. Example branched-chain α-keto acid decarboxylases are known by the EC number 4.1.1.72 and are available from a number of sources, including, but not limited to, *Lactococcus lactis* (GenBank Nos: AAS49166, AY548760; CAG34226, AJ746364, *Salmonella typhimurium* (GenBank Nos: NP_461346, NC_003197), *Clostridium acetobutylicum* (GenBank Nos: NP_149189, NC_001988), *M. caseolyticus,* and *L. grayi.*

The term "branched-chain alcohol dehydrogenase" ("ADH") refers to an enzyme that catalyzes the conversion of isobutyraldehyde to isobutanol. Example branched-chain alcohol dehydrogenases are known by the EC number 1.1.1.265, but may also be classified under other alcohol dehydrogenases (specifically, EC 1.1.1.1 or 1.1.1.2). Alcohol dehydrogenases may be NADPH dependent or NADH dependent. Such enzymes are available from a number of sources, including, but not limited to, *S. cerevisiae* (GenBank Nos: NP_010656, NC_001136, NP_014051, NC_001145), *E. coli* (GenBank Nos: NP_417484, NC_000913), *C. acetobutylicum* (GenBank Nos: NP_349892, NC_003030, NP_349891, NC_003030). U.S. Patent Application Publication No. 2009/0269823 describes SadB, an alcohol dehydrogenase (ADH) from *Achromobacter xylosoxidans.* Alcohol dehydrogenases also include horse liver ADH and *Beijerinkia indica* ADH (as described by U.S. Patent Application Publication No. 2011/0269199).

The term "butanol dehydrogenase" refers to a polypeptide (or polypeptides) having an enzyme activity that catalyzes the conversion of isobutyraldehyde to isobutanol or the conversion of 2-butanone and 2-butanol. Butanol dehydrogenases are a subset of a broad family of alcohol dehydrogenases. Butanol dehydrogenase may be NAD- or NADP-dependent. The NAD-dependent enzymes are known as EC 1.1.1.1 and are available, for example, from *Rhodococcus ruber* (GenBank Nos: CAD36475, AJ491307). The NADP dependent enzymes are known as EC 1.1.1.2 and are available, for example, from *Pyrococcus furiosus* (GenBank Nos: AAC25556, AF013169). Additionally, a butanol dehydrogenase is available from *Escherichia coli* (GenBank Nos: NP 417484, NC_000913) and a cyclohexanol dehydrogenase is available from *Acinetobacter sp.* (GenBank Nos: AAG10026, AF282240). The term "butanol dehydrogenase" also refers to an enzyme that catalyzes the conversion of butyraldehyde to 1-butanol, using either NADH or NADPH as cofactor. Butanol dehydrogenases are available from, for example, C. acetobutylicum (GenBank NOs: NP_149325, NC_001988; note: this enzyme possesses both aldehyde and alcohol dehydrogenase activity); NP_349891, NC_003030; and NP_349892, NC_003030) and *E. coli* (GenBank NOs: NP_417-484, NC_000913).

The term "branched-chain keto acid dehydrogenase" refers to an enzyme that catalyzes the conversion of α-ketoisovalerate to isobutyryl-CoA (isobutyryl-coenzyme A), typically using NAD⁺ (nicotinamide adenine dinucleotide) as an electron acceptor. Example branched-chain keto acid dehydrogenases are known by the EC number 1.2.4.4. Such branched-chain keto acid dehydrogenases are comprised of four subunits and sequences from all subunits are available from a vast array of microorganisms, including, but not limited to, *B. subtilis* (GenBank Nos: CAB14336, Z99116, CAB14335, Z99116, CAB14334, Z99116, CAB14337, Z99116) and *Pseudomonas putida* (GenBank Nos: AAA65614, M57613, AAA65615, M57613, AAA65617, M57613, AAA65618, M57613).

The term "acylating aldehyde dehydrogenase" refers to an enzyme that catalyzes the conversion of isobutyryl-CoA to isobutyraldehyde, typically using either NADH or NADPH as an electron donor. Example acylating aldehyde dehydrogenases are known by the EC numbers 1.2.1.10 and 1.2.1.57. Such enzymes are available from multiple sources, including, but not limited to, *Clostridium beijerinckii* (GenBank Nos: AAD31841, AF157306), C. *acetobutylicum* (GenBank Nos: NP_149325, NC_001988, NP_149199, NC_001988), *P. putida* (GenBank Nos: AAA89106, U13232), and *Thermus thermophilus* (GenBank Nos: YP_145486, NC_006461).

The term "transaminase" refers to an enzyme that catalyzes the conversion of α-ketoisovalerate to L-valine, using either alanine or glutamate as an amine donor. Example transaminases are known by the EC numbers 2.6.1.42 and 2.6.1.66. Such enzymes are available from a number of sources. Examples of sources for alanine-dependent enzymes include, but are not limited to, *E. coli* (GenBank Nos: YP_026231, NC_000913) and *Bacillus licheniformis* (GenBank Nos: YP_093743, NC_006322). Examples of sources for glutamate-dependent enzymes include, but are not limited to, *E. coli* (GenBank Nos: YP_026247, NC_000913), *Saccharomyces cerevisiae* (GenBank Nos: NP_012682, N_001142) and *Methanobacterium thermoautotrophicum* (GenBank Nos: NP_276546, NC_000916).

The term "valine dehydrogenase" refers to an enzyme that catalyzes the conversion of α-ketoisovalerate to L-valine, typically using NAD(P)H as an electron donor and ammonia as an amine donor. Example valine dehydrogenases are known by the EC numbers 1.4.1.8 and 1.4.1.9 and such enzymes are available from a number of sources, including, but not limited to, *Streptomyces coelicolor* (GenBank Nos: NP_628270, NC_003888) and *B. subtilis* (GenBank Nos: CAB14339, Z99116).

The term "valine decarboxylase" refers to an enzyme that catalyzes the conversion of L-valine to isobutylamine and CO₂. Example valine decarboxylases are known by the EC number 4.1.1.14. Such enzymes are found in *Streptomyces,* such as for example, *Streptomyces viridifaciens* (GenBank Nos: AAN10242, AY116644).

The term "omega transaminase" refers to an enzyme that catalyzes the conversion of isobutylamine to isobutyraldehyde using a suitable amino acid as an amine donor. Example omega transaminases are known by the EC number 2.6.1.18 and are available from a number of sources, including, but not limited to, *Alcaligenes denitrificans* (AAP92672, AY330220), *Ralstonia eutropha* (GenBank Nos: YP_294474, NC_007347), *Shewanella oneidensis* (GenBank Nos: NP_719046, NC_004347), and *P. putida* (GenBank Nos: AAN66223, AE016776).

The term "acetyl-CoA acetyltransferase" refers to an enzyme that catalyzes the conversion of two molecules of acetyl-CoA to acetoacetyl-CoA and coenzyme A (CoA). Example acetyl-CoA acetyltransferases are acetyl-CoA acetyltransferases with substrate preferences (reaction in the forward direction) for a short chain acyl-CoA and acetyl-CoA and are classified as E.C. 2.3.1.9 [Enzyme Nomenclature 1992, Academic Press, San Diego]; although, enzymes with a broader substrate range (E.C. 2.3.1.16) will be functional as well. Acetyl-CoA acetyltransferases are available from a number of sources, for example, *Escherichia coli* (GenBank Nos: NP_416728, NC_000913; NCBI (National Center for Biotechnology Information) amino acid sequence, NCBI nucleotide sequence), *Clostridium acetobutylicum* (GenBank Nos: NP_349476.1, NC_003030; NP_149242, NC_001988, *Bacillus subtilis* (GenBank Nos: NP_390297, NC_000964), and *Saccharomyces cerevisiae* (GenBank Nos: NP_015297, N_001148).

The term "3-hydroxybutyryl-CoA dehydrogenase" refers to an enzyme that catalyzes the conversion of acetoacetyl-CoA to 3-hydroxybutyryl-CoA. 3-Example hydroxybutyryl-CoA dehydrogenases may be reduced nicotinamide adenine dinucleotide (NADH)-dependent, with a substrate preference for (S)-3-hydroxybutyryl-CoA or (R)-3-hydroxybutyryl-CoA. Examples may be classified as E.C. 1.1.1.35 and E.C. 1.1.1.30, respectively. Additionally, 3-hydroxybutyryl-CoA dehydrogenases may be reduced nicotinamide adenine dinucleotide phosphate (NADPH)-dependent, with a substrate preference for (S)-3-hydroxybutyryl-CoA or (R)-3-hydroxybutyryl-CoA and are classified as E.C. 1.1.1.157 and E.C. 1.1.1.36, respectively. 3-Hydroxybutyryl-CoA dehydrogenases are available from a number of sources, for example, *C*. *acetobutylicum* (GenBank NOs: NP_349314, NC_003030), *B. subtilis* (GenBank NOs: AAB09614, U29084), *Ralstonia eutropha* (GenBank NOs: YP_294481, NC_007347), and *Alcaligenes eutrophus* (GenBank NOs: AAA21973, J04987).

The term "crotonase" refers to an enzyme that catalyzes the conversion of 3-hydroxybutyryl-CoA to crotonyl-CoA and H₂O. Example crotonases may have a substrate preference for (S)-3-hydroxybutyryl-CoA or (R)-3-hydroxybutyryl-CoA and may be classified as E.C. 4.2.1.17 and E.C. 4.2.1.55, respectively. Crotonases are available from a number of sources, for example, *E. coli* (GenBank NOs: NP_415911, NC_000913), C. *acetobutylicum* (GenBank NOs: NP_349318, NC_003030), *B. subtilis* (GenBank NOs: CAB13705, Z99113), and *Aeromonas caviae* (GenBank NOs: BAA21816, D88825).

The term "butyryl-CoA dehydrogenase" refers to an enzyme that catalyzes the conversion of crotonyl-CoA to butyryl-CoA. Example butyryl-CoA dehydrogenases may be NADH-dependent, NADPH-dependent, or flavin-dependent and may be classified as E.C. 1.3.1.44, E.C. 1.3.1.38, and E.C. 1.3.99.2, respectively. Butyryl-CoA dehydrogenases are available from a number of sources, for example, *C. acetobutylicum* (GenBank NOs: NP_347102, NC_ 003030), *Euglena gracilis* (GenBank NOs: Q5EU90), AY741582), *Streptomyces collinus* (GenBank NOs: AAA92890, U37135), and *Streptomyces coelicolor* (GenBank NOs: CAA22721, AL939127).

The term "butyraldehyde dehydrogenase" refers to an enzyme that catalyzes the conversion of butyryl-CoA to butyraldehyde, using NADH or NADPH as cofactor. Butyraldehyde dehydrogenases with a preference for NADH are known as E.C. 1.2.1.57 and are available from, for example, *Clostridium beijerinckii* (GenBank NOs: AAD31841, AF157306) and *C. acetobutylicum* (GenBank NOs: NP.sub.--149325, NC.sub.--001988).

The term "isobutyryl-CoA mutase" refers to an enzyme that catalyzes the conversion of butyryl-CoA to isobutyryl-CoA. This enzyme uses coenzyme B₁₂ as cofactor. Example isobutyryl-CoA mutases are known by the EC number 5.4.99.13. These enzymes are found in a number of *Streptomyces,* including, but not limited to, *Streptomyces cinnamonensis* (GenBank Nos: AAC08713, U67612, CAB59633, AJ246005), *S. coelicolor* (GenBank Nos: CAB70645, AL939123, CAB92663, AL939121), and *Streptomyces avermitilis* (GenBank Nos: NP_824008, NC_003155, NP_824637, NC_003155).

The term "acetolactate decarboxylase" refers to a polypeptide (or polypeptides) having an enzyme activity that catalyzes the conversion of alpha-acetolactate to acetoin. Example acetolactate decarboxylases are known as EC 4.1.1.5 and are available, for example, from *Bacillus subtilis* (GenBank Nos: AAA22223, L04470), *Klebsiella terrigena* (GenBank Nos: AAA25054, L04507) and *Klebsiella pneumoniae* (GenBank Nos: AAU43774, AY722056).

The term "acetoin aminase" or "acetoin transaminase" refers to a polypeptide (or polypeptides) having an enzyme activity that catalyzes the conversion of acetoin to 3-amino-2-butanol. Acetoin aminase may utilize the cofactor pyridoxal 5'-phosphate or NADH (reduced nicotinamide adenine dinucleotide) or NADPH (reduced nicotinamide adenine dinucleotide phosphate). The resulting product may have (R) or (S) stereochemistry at the 3-position. The pyridoxal phosphate-dependent enzyme may use an amino acid such as alanine or glutamate as the amino donor. The NADH- and NADPH-dependent enzymes may use ammonia as a second substrate. A suitable example of an NADH dependent acetoin aminase, also known as amino alcohol dehydrogenase, is described by Ito, et al. (U.S. Patent No. 6,432,688). An example of a pyridoxal-dependent acetoin aminase is the amine:pyruvate aminotransferase (also called amine:pyruvate transaminase) described by Shin and Kim (J. Org. Chem. 67:2848-2853, 2002).

The term "acetoin kinase" refers to a polypeptide (or polypeptides) having an enzyme activity that catalyzes the conversion of acetoin to phosphoacetoin. Acetoin kinase may utilize ATP (adenosine triphosphate) or phosphoenolpyruvate as the phosphate donor in the reaction. Enzymes that catalyze the analogous reaction on the similar substrate dihydroxyacetone, for example, include enzymes known as EC 2.7.1.29 (Garcia-Alles, et al., Biochemistry 43:13037-13046, 2004).

The term "acetoin phosphate aminase" refers to a polypeptide (or polypeptides) having an enzyme activity that catalyzes the conversion of phosphoacetoin to 3-amino-2-butanol O-phosphate. Acetoin phosphate aminase may use the cofactor pyridoxal 5'-phosphate, NADH or NADPH. The resulting product may have (R) or (S) stereochemistry at the 3-position. The pyridoxal phosphate-dependent enzyme may use an amino acid such as alanine or glutamate. The NADH and NADPH-dependent enzymes may use ammonia as a second substrate. Although there are no reports of enzymes catalyzing this reaction on phosphoacetoin, there is a pyridoxal phosphate-dependent enzyme that is proposed to carry out the analogous reaction on the similar substrate serinol phosphate (Yasuta, et al., Appl. Environ. Microbial. 67:4999-5009, 2001).

The term "aminobutanol phosphate phospholyase," also called "amino alcohol O-phosphate lyase," refers to a polypeptide (or polypeptides) having an enzyme activity that catalyzes the conversion of 3-amino-2-butanol O-phosphate to 2-butanone. Amino butanol phosphate phospho-lyase may utilize the cofactor pyridoxal 5'-phosphate. There are reports of enzymes that catalyze the analogous reaction on the similar substrate 1-amino-2-propanol phosphate (Jones, et al., Biochem J. 134:167-182, 1973). U.S. Patent Application Publication No. 2007/0259410 describes an aminobutanol phosphate phospho-lyase from the organism *Erwinia carotovora.*

The term "aminobutanol kinase" refers to a polypeptide (or polypeptides) having an enzyme activity that catalyzes the conversion of 3-amino-2-butanol to 3-amino-2butanol O-phosphate. Amino butanol kinase may utilize ATP as the phosphate donor. Although there are no reports of enzymes catalyzing this reaction on 3-amino-2-butanol, there are reports of enzymes that catalyze the analogous reaction on the similar substrates ethanolamine and 1-amino-2-propanol (Jones, et al., supra). U.S. Patent Application Publication No. 2009/0155870 describes, in Example 14, an amino alcohol kinase of *Erwinia carotovora subsp. Atroseptica.*

The term "butanediol dehydrogenase" also known as "acetoin reductase" refers to a polypeptide (or polypeptides) having an enzyme activity that catalyzes the conversion of acetoin to 2,3-butanediol. Butanedial dehydrogenases are a subset of the broad family of alcohol dehydrogenases. Butanediol dehydrogenase enzymes may have specificity for production of (R)- or (S)-stereochemistry in the alcohol product. (S)-specific butanediol dehydrogenases are known as EC 1.1.1.76 and are available, for example, from *Klebsiella pneumoniae* (GenBank Nos: BBA13085, D86412). (R)-specific butanediol dehydrogenases are known as EC 1.1.1.4 and are available, for example, from *Bacillus cereus* (GenBank Nos. NP 830481, NC_004722; AAP07682, AE017000), and *Lactococcus lactis* (GenBank Nos. AAK04995, AE006323).

The term "butanediol dehydratase," also known as "dial dehydratase" or "propanediol dehydratase" refers to a polypeptide (or polypeptides) having an enzyme activity that catalyzes the conversion of 2,3-butanediol to 2-butanone. Butanediol dehydratase may utilize the cofactor adenosyl cobalamin (also known as coenzyme Bw or vitamin B12; although vitamin B12 may refer also to other forms of cobalamin that are not coenzyme B12). Adenosyl cobalamin-dependent enzymes are known as EC 4.2.1.28 and are available, for example, from *Klebsiella oxytoca* (GenBank Nos: AA08099 (alpha subunit), D45071; BAA08100 (beta subunit), D45071; and BBA08101 (gamma subunit), D45071 (Note all three subunits are required for activity)], and *Klebsiella pneumonia* (GenBank Nos: AAC98384 (alpha subunit), AF102064; GenBank Nos: AAC98385 (beta subunit), AF102064, GenBank Nos: AAC98386 (gamma subunit), AF102064). Other suitable dial dehydratases include, but are not limited to, B12-dependent dial dehydratases available from *Salmonella typhimurium* (GenBank Nos: AAB84102 (large subunit), AF026270; GenBank Nos: AAB84103 (medium subunit), AF026270; GenBank Nos: AAB84104 (small subunit), AF026270); and *Lactobacillus collinoides* (GenBank Nos: CAC82541 (large subunit), AJ297723 ; GenBank Nos: CAC82542 (medium subunit); AJ297723; GenBank Nos: CAD01091 (small subunit), AJ297723); and enzymes from *Lactobacillus brevis* (particularly strains CNRZ 734 and CNRZ 735, Speranza, et al., J. Agric. Food Chem. 45:3476-3480, 1997), and nucleotide sequences that encode the corresponding enzymes. Methods of dial dehydratase gene isolation are well known in the art (e.g., U.S. Patent No. 5,686,276).

The term "pyruvate decarboxylase" refers to an enzyme that catalyzes the decarboxylation of pyruvic acid to acetaldehyde and carbon dioxide. Pyruvate dehydrogenases are known by the EC number 4.1.1.1. These enzymes are found in a number of yeast, including *Saccharomyces cerevisiae* (GenBank Nos: CAA97575, CAA97705, CAA97091).

It will be appreciated that host cells comprising an isobutanol biosynthetic pathway as provided herein may further comprise one or more additional modifications. U.S. Patent Application Publication No. 2009/0305363 discloses increased conversion of pyruvate to acetolactate by engineering yeast for expression of a cytosol-localized acetolactate synthase and substantial elimination of pyruvate decarboxylase activity. In some embodiments, the host cells comprise modifications to reduce glycerol-3-phosphate dehydrogenase activity and/or disruption in at least one gene encoding a polypeptide having pyruvate decarboxylase activity or a disruption in at least one gene encoding a regulatory element controlling pyruvate decarboxylase gene expression as described in U.S. Patent Application Publication No. 2009/0305363 (incorporated herein by reference), modifications to a host cell that provide for increased carbon flux through an Entner-Doudoroff Pathway or reducing equivalents balance as described in U.S. Patent Application Publication No. 2010/0120105. Other modifications include integration of at least one polynucleotide encoding a polypeptide that catalyzes a step in a pyruvate-utilizing biosynthetic pathway. Other modifications include at least one deletion, mutation, and/or substitution in an endogenous polynucleotide encoding a polypeptide having acetolactate reductase activity. Additional modifications include a deletion, mutation, and/or substitution in an endogenous polynucleotide encoding a polypeptide having aldehyde dehydrogenase and/or aldehyde oxidase activity. In some embodiments, the polypeptide having aldehyde dehydrogenase activity is *ALD6* from *Saccharomyces cerevisiae* or a homolog thereof. A genetic modification which has the effect of reducing glucose repression wherein the yeast production host cell is pdc- is described in U.S. Patent Application Publication No. 2011/0124060, incorporated herein by reference. In some embodiments, the pyruvate decarboxylase that is deleted or down-regulated is selected from the group consisting of: *PDC1*, *PDC5*, *PDC6,* and combinations thereof. In some embodiments, host cells contain a deletion or down-regulation of a polynucleotide encoding a polypeptide that catalyzes the conversion of glyceraldehyde-3-phosphate to glycerate 1,3, bisphosphate. In some embodiments, the enzyme that catalyzes this reaction is glyceraldehyde-3-phosphate dehydrogenase.

Certain suitable proteins having the ability to catalyze indicated substrate to product conversions are described herein and other suitable proteins are provided in the art. For example, U.S. Patent Application Publication Nos. 2008/0261230, 2009/0163376, and 2010/0197519 describe acetohydroxy acid isomeroreductases; U.S. Patent Application Publication No. 2010/0081154 describes dihydroxyacid dehydratases; an alcohol dehydrogenase is described in U.S. Patent Application Publication No. 2009/0269823.

It is well understood by one skilled in the art that many levels of sequence identity are useful in identifying polypeptides from other species, wherein such polypeptides have the same or similar function or activity and are suitable for use in the recombinant microorganisms described herein. Useful examples of percent identities include, but are not limited to, 75%, 80%, 85%, 90%, or 95%, or any integer percentage from 75% to 100% can be useful in describing the present invention such as 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99%.

Standard recombinant DNA and molecular cloning techniques used here are well known in the art and are described by Sambrook, J., Fritsch, E. F. and Maniatis, T., Molecular Cloning: A Laboratory Manual, Second Edition, Cold Spring Harbor Laboratory Press, Cold Spring Harbor, NY (1989) (hereinafter "Maniatis"); and by Silhavy, T. J., Bennan, M. L. and Enquist, L. W., Experiments with Gene Fusions, Cold Spring Harbor Laboratory Press, Cold Spring Harbor, NY (1984); and by Ausubel, F. M. et al., Current Protocols in Molecular Biology, published by Greene Publishing Assoc. and Wiley-Interscience (1987). Additional methods used here are in Methods in Enzymology, Volume 194, Guide to Yeast Genetics and Molecular and Cell Biology (Part A, 2004, Christine Guthrie and Gerald R. Fink (Eds.), Elsevier Academic Press, San Diego, CA).

Methods for increasing or for reducing gene expression of the target genes above are well known to one skilled in the art. Methods for gene expression in yeasts are known in the art as described, for example, in Methods in Enzymology, Volume 194, Guide to Yeast Genetics and Molecular and Cell Biology (Part A, 2004, Christine Guthrie and Gerald R. Fink (Eds.), Elsevier Academic Press, San Diego, Calif.). For example, methods for increasing expression include increasing the number of genes that are integrated in the genome or on plasmids that express the target protein, and using a promoter that is more highly expressed than the natural promoter. Promoters that may be operably linked in a constructed chimeric gene for expression include, for example, constitutive promoters FBA1, TDH3, ADH1, and GPM1, and the inducible promoters GAL1, GAL10, and CUP1. Suitable transcriptional terminators that may be used in a chimeric gene construct for expression include, but are not limited to FBA1t, TDH3t, GPM1t, ERG10t, GAL1t, CYC1t, and ADH1t.

Suitable promoters, transcriptional terminators, and coding regions may be cloned into *E.* coli-yeast shuttle vectors, and transformed into yeast cells. These vectors allow for propagation in both *E. coli* and yeast strains. Typically, the vector contains a selectable marker and sequences allowing autonomous replication or chromosomal integration in the desired host. Plasmids used in yeast are, for example, shuttle vectors pRS423, pRS424, pRS425, and pRS426 (American Type Culture Collection, Rockville, Md.), which contain an *E. coli* replication origin (e.g., pMB1), a yeast 2µ origin of replication, and a marker for nutritional selection. The selection markers for these four vectors are HIS3 (vector pRS423), TRP1 (vector pRS424), LEU2 (vector pRS425) and URA3 (vector pRS426). Construction of expression vectors may be performed by either standard molecular cloning techniques in *E. coli* or by the gap repair recombination method in yeast.

While various embodiments of the present invention have been described above, it should be understood that they have been presented by way of example only, and not limitation. It will be apparent to persons skilled in the relevant art that various changes in form and detail can be made. Thus, the breadth and scope of the present invention should not be limited by any of the above-described exemplary embodiments, but should be defined only in accordance with the claims.

All publications, patents, and patent applications mentioned in this specification are indicative of the level of skill of those skilled in the art to which this invention pertains.

### EXAMPLES

The following non-limiting examples will further illustrate the invention. It should be understood that, while the following examples involve corn as feedstock, other biomass sources can be used for feedstock.

As used herein, the meaning of abbreviations used was as follows: "g" means gram(s), "kg" means kilogram(s), "L" means liter(s), "mL" means milliliter(s), "mL/L" means milliliter(s) per liter, "mL/min" means milliliter(s) per min, "DI" means deionized, "uM" means micrometer(s), "nm" means nanometer(s), "w/v" means weight/volume, "rpm" means revolutions per minute, "°C" means degree(s) Celsius, and "slpm" means standard liter(s) per minute. The foregoing description of the specific embodiments will so fully reveal the general nature of the invention that others can, by applying knowledge within the skill of the art, readily modify and/or adapt for various applications such specific embodiments, without undue experimentation, without departing from the general concept of the present disclosure.

### EXAMPLE 1

### Preparation of Corn Mash

Approximately 100 kg of liquefied corn mash was prepared in three equivalent batches using a 30 L glass, jacketed resin kettle. The kettle was set up with mechanical agitation, temperature control, and pH control. The protocol used for all three batches was as follows: (a) mixing ground corn with tap water (30 wt% corn on a dry basis), (b) heating the slurry to 55°C while agitating, (c) adjusting pH of the slurry to 5.8 with either NaOH or H₂SO₄, (d) adding alpha-amylase (0.02 wt% on a dry corn basis), (e) heating the slurry to 85°C, (f) adjusting pH to 5.8, (g) holding the slurry at 85°C for 2 hrs while maintaining pH at 5.8, and (h) cooling the slurry to 25°C.

The corn used was whole kernel yellow corn from Pioneer (3335). It was ground in a hammer-mill using a 1 mm screen. The moisture content of the ground corn was measured to be 12 wt%, and the starch content of the ground corn was measured to be 71.4 wt% on a dry corn basis. The alpha-amylase enzyme was Liquozyme® SC DS available from Novozymes (Franklinton, NC). The total amounts of the ingredients used for all three batches combined were: 33.9 kg of ground corn (12% moisture), 65.4 kg of tap water, and 0.006 kg of Liquozyme® SC DS. A total of 0.297 kg of NaOH (17 wt%) was added to control pH. No H₂SO₄ was required. The total amount of liquefied corn mash recovered from the three 30 L batches was 99.4 kg.

### EXAMPLE 2

### Solids Removal

The solids were removed from the mash produced in Example 1 by centrifugation in a large floor centrifuge which contained six 1 L bottles. 73.4 kg of mash was centrifuged at 8000 rpm for 20 min at 25°C yielding 44.4 kg of centrate and 26.9 kg of wet cake. It was determined that the centrate contained <1 wt% suspended solids, and that the wet cake contained approximately 18 wt% suspended solids. This implies that the original liquefied mash contained approximately 7 wt% suspended solids. This is consistent with the corn loading and starch content of the corn used assuming most of the starch was liquefied. If all of the starch was liquefied, the 44.4 kg of centrate recovered directly from the centrifuge would have contained approximately 23 wt% dissolved oligosaccharides (liquefied starch). About 0.6 kg of isobutanol was added to 35.4 kg of centrate to preserve it. The resulting 36.0 kg of centrate, which contained 1.6 wt% isobutanol, was used as a stock solution.

### EXAMPLE 3

### Removal of Corn Oil by Removing Undissolved Solids

This example demonstrates the potential to remove and recover corn oil from corn mash by removing the undissolved solids prior to fermentation. The effectiveness of the extraction solvent can be compromised if it is diluted with corn oil. Reduction in solvent partition coefficient must be minimized in an extractive fermentation process in order for liquid-liquid extraction to be a viable separation method for practicing in situ product removal (ISPR).

Approximately 1000 g of liquefied corn mash was prepared in a 1 L glass, jacketed resin kettle. The kettle was set up with mechanical agitation, temperature control, and pH control. The following protocol was used: mixed ground corn with tap water (26 wt% corn on a dry basis), heated the slurry to 55 °C while agitating, adjusted pH to 5.8 with either NaOH or H₂SO₄, added alpha-amylase (0.02 wt% on a dry corn basis), continued heating to 85 °C, adjusted pH to 5.8, held at 85 °C for 2 hrs while maintaining pH at 5.8, cool to 25 °C. The corn used was whole kernel yellow corn from Pioneer (3335). It was ground in a hammer-mill using a 1 mm screen. The moisture content of the ground corn was measured to be about 11.7 wt%, and the starch content of the ground corn was measured to be about 71.4 wt% on a dry corn basis. The alpha-amylase enzyme was Liquozyme® SC DS from Novozymes (Franklinton, NC). The total amounts of the ingredients used were: 294.5 g of ground corn (11.7% moisture), 705.5 g of tap water, and 0.059 g of Liquozyme® SC DS. N₂0 (4.3 g) was added to dilute the enzyme, and a total of 2.3 g of 20% NaOH solution was added to control pH. About 952 g of mash was recovered. Note that there were losses due to mash sticking on walls of kettle and CF bottles.

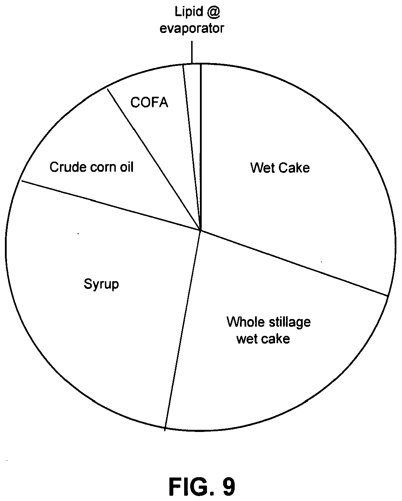

The liquefied corn mash was centrifuged at 5000 rpm (7260 g's) for 30 minutes at 40 °C to remove the undissolved solids from the aqueous solution of oligosaccharides. Removing the solids by centrifugation also resulted in the removal of free corn oil as a separate organic liquid layer on top of the aqueous phase, as shown, for example, in FIG. 12. Approximately 1.5 g of corn oil was recovered from the organic layer floating on top of the aqueous phase shown in Figure 9. It was determined by hexane extraction that the ground corn used to produce the liquefied mash contained about 3.5 wt% corn oil on a dry corn basis. This corresponds to about 9 g of corn oil fed to the liquefaction process with the ground corn.

Approximately 1 g of corn oil was recovered from the organic layer floating on top of the aqueous phase. About 617 g of liquefied starch solution was recovered leaving about 334 g of wet cake. The wet cake contained most of the undissolved solids that were in the liquefied mash. The liquefied starch solution contained about 0.2 wt% undissolved solids. The wet cake contained about 21 wt% undissolved solids. The wet cake was washed with 1000 g of tap water to remove the oligosaccharides still in the cake. This was done by mixing the cake with the water to form a slurry. The slurry was then centrifuged under the same conditions used to centrifuge the original mash in order to recover the washed solids. Removing the washed solids by centrifugation also resulted in the removal of some additional free corn oil as a separate organic liquid layer on top of the aqueous phase. Corn oil was recovered from the organic layer floating on top of the aqueous phase.

The wet solids were washed two more times using a 1000 g of tap water each time to remove essentially all of the liquefied starch. The final washed solids were dried in a vacuum oven overnight at 80 °C and about 20 inches Hg vacuum. The amount of corn oil remaining in the dry solids, presumably still in the germ, was determined by hexane extraction. It was measured that a 3.60 g sample of relatively dry solids (about 2 wt% moisture) contained 0.22 g of corn oil. This result corresponds to 0.0624 g corn oil/g dry solids. This was for washed solids which means there are no residual oligosaccharides in the wet solids. After centrifuging the liquefied corn mash to separate the layer of free corn oil and the aqueous solution of oligosaccharides from the wet cake, it was determined that about 334 g of wet cake containing about 21 wt% undissolved solids remained. This corresponds to the wet cake comprising about 70.1 g of undissolved solids. At 0.0624 g corn oil/g dry solids, the solids in the wet cake should contain about 4.4 g of corn oil.

### EXAMPLE 4

### General Methods for Fermentation

### Seed Flask Growth

A *Saccharomyces cerevisiae* strain that was engineered to produce isobutanol from a carbohydrate source, with pdc1 deleted, pdc5 deleted, and pdc6 deleted was grown to 0.55-1.1 g/L dcw (OD600 1.3-2.6 - Thermo Helios α Thermo Fisher Scientific Inc., Waltham, Massachusetts) in seed flasks from a frozen culture. The culture was grown at 26 °C in an incubator rotating at 300 rpm. The frozen culture was previously stored at - 80 °C. The composition of the first seed flask medium was:
3.0 g/L dextrose
3.0 g/L ethanol, anhydrous
3.7 g/L ForMedium™ Synthetic Complete Amino Acid (Kaiser) Drop-Out: without HIS, without URA (Reference No. DSCK162CK)
6.7 g/L Difco Yeast Nitrogen Base without amino acids (No. 291920).

Twelve milliliters from the first seed flask culture was transferred to a 2 L flask and grown at 30 °C in an incubator rotating at 300 rpm. The second seed flask has 220 mL of the following medium:
30.0 g/L dextrose
   5.0 g/L ethanol, anhydrous
   3.7 g/L ForMedium™ Synthetic Complete Amino Acid (Kaiser) Drop-Out: without HIS, without URA (Reference No. DSCK162CK)
   6.7 g/L Difco Yeast Nitrogen Base without amino acids (No. 291920)
   0.2M MES Buffer titrated to pH 5.5-6.0.

The culture was grown to 0.55-1.1 g/L dcw (OD600 1.3-2.6). An addition of 30 mL of a solution containing 200 g/L peptone and 100 g/L yeast extract was added at this cell concentration. Then an addition of 300 mL of 0.2 uM filter sterilized Cognis, 90-95% oleyl alcohol was added to the flask. The culture continues to grow to > 4 g/L dcw (OD600 > 10) before being harvested and added to the fermentation.

### Fermentation Preparation

### Initial Fermentor Preparation

A glass jacked, 2 L fermentor (Sartorius AG, Goettingen, Germany) was charged with liquefied mash either with or without solids (centrate). A pH probe (Hamilton Easyferm Plus K8, part number: 238627, Hamilton Bonaduz AG, Bonaduz, Switzerland) was calibrated through the Sartorius DCU-3 Control Tower Calibration menu. The zero was calibrated at pH=7. The span was calibrated at pH=4. The probe was then placed into the fermentor, through the stainless steel head plate. A dissolved oxygen probe (pO2 probe) was also placed into the fermentor through the head plate. Tubing used for delivering nutrients, seed culture, extracting solvent, and base were attached to the head plate and the ends were foiled. The entire fermentor was placed into a Steris (Steris Corporation, Mentor, Ohio) autoclave and sterilized in a liquid cycle for 30 minutes.

The fermentor was removed from the autoclave and placed on a load cell. The jacket water supply and return line was connected to the house water and clean drain, respectively. The condenser cooling water in and water out lines were connected to a 6-L recirculating temperature bath running at 7°C. The vent line that transfers the gas from the fermentor was connected to a transfer line that was connected to a Thermo mass spectrometer (Prima dB, Thermo Fisher Scientific Inc., Waltham, Massachusetts). The sparger line was connected to the gas supply line. The tubing for adding nutrients, extract solvent, seed culture, and base was plumbed through pumps or clamped closed. The autoclaved material, 0.9% w/v NaCl was drained prior to the addition of liquefied mash.

### Lipase Treatment Post-Liquefaction

The fermentor temperature was set to 55°C instead of 30°C after the liquefaction cycle was complete (Liquefaction). The pH was manually controlled at pH=5.8 by making bolus additions of acid or base when needed. A lipase enzyme stock solution was added to the fermentor to a final lipase concentration of 10 ppm. The fermentor was held at 55°C, 300 rpm, and 0.3 slpm N₂ overlay for >6 hrs. After the lipase treatment was complete the fermentor temperature was set to 30°C.

### Nutrient Addition Prior to Inoculation

Added 7.0 mL/L (post-inoculation volume) of ethanol (200 proof, anhydrous) just prior to inoculation. Add thiamine to 20 mg/L final concentration just prior to inoculation. Add 100 mg/L nicotinic acid just prior to inoculation.

### Fermentor Inoculation

The fermentors pO₂ probe was calibrated to zero while N₂ was being added to the fermentor. The fermentors pO₂ probe was calibrated to its span with sterile air sparging at 300 rpm. The fermentor was inoculated after the second seed flask was > 4 g/L dcw. The shake flask was removed from the incubator/shaker for 5 minutes allowing a phase separation of the oleyl alcohol phase and the aqueous phase. The 55 mL of the aqueous phase was transferred to a sterile, inoculation bottle. The inoculum was pumped into the fermentor through a peristaltic pump.

### Oleyl Alcohol or Corn Oil Fatty Acids Addition After Inoculation

Added 1 L/L (post-inoculation volume) of oleyl alcohol or corn oil fatty acids immediately after inoculation.

### Fermentor Operating Conditions

The fermentor was operated at 30°C for the entire growth and production stages. The pH was allowed to drop from a pH between 5.7-5.9 to a control set-point of 5.2 without adding any acid. The pH was controlled for the remainder of the growth and production stage at a pH =5.2 with ammonium hydroxide. Sterile air was added to the fermentor, through the sparger, at 0.3 slpm for the remainder of the growth and production stages. The pO₂ was set to be controlled at 3.0% by the Sartorius DCU-3 Control Box PID control loop, using stir control only, with the stirrer minimum being set to 300 rpm and the maximum being set to 2000 rpm. The glucose was supplied through simultaneous saccharification and fermentation of the liquefied corn mash by adding a α-amylase (glucoamylase). The glucose was kept excess (1-50 g/L) for as long as starch was available for saccharification.

### EXAMPLE 5

### Wet Cake Generated from the Removal of Solids from Liquefied Corn Mash

This example demonstrated the recovery of a wet cake and recovery of starches from a wet cake by washing the cake twice with water, where the cake was generated by centrifuging liquefied mash. Liquefied corn mash was fed to a continuous decanter centrifuge to produce a centrate stream (C-1) and a wet cake (WC-1). The centrate was a relatively solids-free, aqueous solution of soluble starch, and the wet cake was concentrated in solids compared to the feed mash. A portion of the wet cake was mixed with hot water to form a slurry (S-1). The slurry was pumped back through the decanter centrifuge to produce a wash water centrate (C-2) and a washed wet cake (WC-2). C-2 was a relatively solids-free, dilute aqueous solution of soluble starch. The concentration of soluble starch in C-2 was less than the concentration of soluble starch in the centrate produced from the separation of mash. The liquid phase held up in WC-2 was more dilute in starch than the liquid in the wet cake produced from the separation of mash. The washed wet cake (WC-2) was mixed with hot water to form a slurry (S-2). The ratio of water charged to the amount of soluble starch in the wet cake charged was the same in both wash steps. The second wash slurry was pumped back through the decanter centrifuge to produce a second wash water centrate (C-3) and a wet cake (WC-3) that had been washed twice. C-3 was a relatively solids-free, dilute aqueous solution of soluble starch. The concentration of soluble starch in C-3 was less than the concentration of soluble starch in the centrate produced in the first wash stage (C-2), and thus the liquid phase held up in WC-3 (second washed wet cake) was more dilute in starch than in WC-2 (first washed wet cake). The total soluble starch in the two wash centrates (C-2 and C-3) is the starch that was recovered and could be recycled back to liquefaction. The soluble starch in the liquid held up in the final washed wet cake is much less that in the wet cake produced in the original separation of the mash.

### Production of Liquefied Corn Mash

Approximately 1000 gallons of liquefied corn mash was produced in a continuous dry-grind liquefaction system consisting of a hammer mill, slurry mixer, slurry tank, and liquefaction tank. Ground corn, water, and alpha-amylase were fed continuously. The reactors were outfitted with mechanical agitation, temperature control, and pH control using either ammonia or sulfuric acid. The reaction conditions were as follows:

| | |
|---|---|
| Hammer mill screen size: | 7/64" |

### Feed Rates to Slurry Mixer

| | |
|---|---|
| - Ground Corn: | 560 lbm/hr (14.1 wt% moisture) |
| - Process Water: | 16.6 lbm/min (200 F) |
| - Alpha-Amylase: | 61 g/hr (Genecor: Spezyme® ALPHA) |

### Slurry Tank Conditions:

| | |
|---|---|
| - Temperature: | 185 °F (85 °C) |
| - pH: | 5.8 |
| - Residence Time: | 0.5 hr |
| - Dry Corn Loading: | 31 wt% |
| - Enzyme Loading: | 0.028 wt% (dry corn basis) |

### Liquefaction Tank Conditions:

| | |
|---|---|
| - Temperature: | 185 °F (85 °C) |
| - pH: | 5.8 |
| - Residence Time: | about 3 hrs |
| - No additional enzyme added. | |

The production rate of liquefied corn mash was about 3 gpm. The starch content of the ground corn was measured to be about 70 wt% on a dry corn basis. The total solids (TS) of the liquefied mash was about 31 wt%, and the total suspended solids (TSS) was approximately 7 wt%. The liquid phase contained about 23-24 wt% liquefied starch as measured by HPLC (soluble oligosaccharides).

The liquefied mash was centrifuged in a continuous decanter centrifuge (make, model) at the following conditions:

| | |
|---|---|
| Bowl Speed: | 5000 rpm (about 3600 g's) |
| Differential Speed: | 15 rpm |
| Weir Diameter: | 185 mm (weir plate removed) |
| Feed Rate: | Varied from 5-20 gpm. |

Approximately 850 gal of centrate and approximately 1400 lbm of wet cake were produced by centrifuging the mash. The total solids in the wet cake were measured to be about 46.3% (suspended + dissolved) by moisture balance. Knowing that the liquid phase contained about 23 wt% soluble starch, it was estimated that the total suspended solids in the wet cake was about 28 wt%. It was estimated that the wet cake contained approximately 12% of the soluble starch that was present in the liquefied mash prior to the centrifuge operation.

### EXAMPLE 6

### Lipid Analysis

Lipid analysis was conducted by conversion of the various fatty acid-containing compound classes to fatty acid methyl esters ("FAMEs") by transesterification. Glycerides and phospholipids were transesterified using sodium methoxide in methanol. Glycerides, phospholipids, and free fatty acids were transesterified using acetyl chloride in methanol. The resulting FAMEs were analyzed by gas chromatography using an Agilent 7890 GC fitted with a 30-m X 0.25 mm (i.d.) OMEGAWAX™ (Supelco, SigmaAldrich, St. Louis, MO) column after dilution in toluene/hexane (2:3). The oven temperature was increased from 160°C to 200 °C at 5°C/min then 200°C to 250°C (hold for 10 min) at 10°C/min. FAME peaks recorded via GC analysis were identified by their retention times, when compared to that of known methyl esters (MEs), and quantitated by comparing the FAME peak areas with that of the internal standard (C15:0 triglyceride, taken through the transesterification procedure with the sample) of known amount. Thus, the approximate amount (mg) of any fatty acid FAME ("mg FAME") is calculated according to the formula: (area of the FAME peak for the specified fatty acid/ area of the 15:0 FAME peak) * (mg of the internal standard C15:0 FAME). The FAME result can then be corrected to mg of the corresponding fatty acid by dividing by the appropriate molecular weight conversion factor of 1.052. All internal and reference standards are obtained from Nu-Chek Prep, Inc.

The fatty acid results obtained for samples transesterified using sodium methoxide in methanol are converted to the corresponding triglyceride levels by multiplying the molecular weight conversion factor of 1.045. Triglycerides generally account for approximately 80% to 90% of the glycerides in the samples studies for this example, with the remainder being diglycerides. Monoglyceride and phospholipid contents are generally negligible. The total fatty acid results obtained for a sample transesterified using acetyl chloride in methanol are corrected for glyceride content by subtracting the fatty acids determined for the same sample using the sodium methoxide procedure. The result is the free fatty acid content of the sample.

The distribution of the glyceride content (monoglycerides, diglycerides, triglycerides, and phospholipids) is determined using thin layer chromatography. A solution of the oil dissolved in 6:1 chloroform/methanol is spotted near the bottom of a glass plate precoated with silica gel. The spot is then chromatographed up the plate using a 70:30:1 hexane/diethyl ether/acetic acid solvent system. Separated spots corresponding to monoglycerides, diglycerides, triglycerides, and phospholipids are then detected by staining the plate with iodine vapor. The spots are then scraped off the plate, transesterified using the acetyl chloride in methanol procedure, and analyzed by gas chromatography. The ratios of the totaled peak areas for each spot to the totaled peak areas for all the spots are the distribution of the various glycerides.

### EXAMPLE 7

### Solids from stillage and extraction by desolventizer to recover fatty acids, esters, and triglycerides

This example illustrated the removal of solids from stillage and extraction by desolventizer to recover fatty acids, esters, and triglycerides from the solids. During fermentation, solids are separated from whole stillage and fed to a desolventizer where they are contacted with 1.1 tons/hr of steam. The flow rates for the whole stillage wet cake (extractor feed), solvent, the extractor miscella, and extractor discharge solids are as shown in Table 1. Table values are short tons/hr.

**Table 1**

| | Solids from whole stillage | Solvent | Miscella | Extractor discharge solids |
|---|---|---|---|---|
| Fatty acids | 0.099 | 0 | 0.0982 | 0.001 |
| Undissolved solids | 17.857 | 0 | 0.0009 | 17.856 |
| Fatty acid butyl esters | 2.866 | 0 | 2.837 | 0.0287 |
| Hexane | 0 | 11.02 | 10.467 | 0.555 |
| Triglyceride | 0.992 | 0 | 0.982 | 0.0099 |
| Water | 29.762 | 0 | 29.464 | 0.297 |

Solids exiting the desolventizer are fed to a dryer. The vapor exiting the desolventizer contains 0.55 tons/hr of hexane and 1.102 tons/hr of water. This stream is condensed and fed to a decanter. The water-rich phase exiting the decanter contains about 360 ppm of hexane. This stream is fed to a distillation column where the hexane is removed from the water-rich stream. The hexane enriched stream exiting the top of the distillation column is condensed and fed to the decanter. The organic-rich stream exiting the decanter is fed to a distillation column. Steam (11.02 tons/hr) is fed to the bottom of the distillation column. The composition of the overhead and bottom products for this column are shown in Table 2. Table values are tons/hr.

**Table 2**

| | **Bottoms** | **Overheads** |
|---|---|---|
| Fatty acids | 0.0981 | 0 |
| Fatty acid butyl esters | 2.8232 | 0 |
| Hexane | 0.0011 | 11.12 |
| Triglyceride | 0.9812 | 0 |
| Water | 0 | 11.02 |

### EXAMPLE 8

### Recovery of by-products from mash

This example illustrates the recovery of by-products from mash. Corn oil separated from mash under the conditions described in Example 3 with the exception that a tricanter centrifuge (Flottweg Z23-4 bowl diameter, 230 mm, length to diameter ratio 4:1) was used with these conditions:

| | |
|---|---|
| Bowl Speed: | 5000 rpm |
| Differential Speed: | 10 rpm |
| Feed Rate: | 3 gpm |
| Phase Separator Disk: | 138 mm |
| Impeller Setting: | 144 mm. |

The corn oil separate had 81% triglycerides, 6% free fatty acids, 4% diglyceride, and 5% total of phospholipids and monoglycerides as determined by the methods described in Example 6 and thin layer chromatography.

The solids separated from mash under the conditions described above had a moisture content of 58% as determined by weight loss upon drying and had 1.2% triglycerides and 0.27% free fatty acids as determined by the method described in Example 6.

The composition of solids separated from whole stillage, oil extracted between evaporator stages, by-product extractant and Condensed Distillers Solubles (CDS) in Table 5 were calculated assuming the composition of whole stillage shown in Table 3 and the assumptions in Table 3 (separation at tricanter centrifuge. The values of Table 2 were obtained from an Aspen Plus® model (Aspen Technology, Inc., Burlington, MA). This model assumes that corn oil is not extracted from mash. It is estimated that the protein content on a dry basis of cells, dissolved solids, and suspended solids is approximately 50%, 22%, and 35.5%, respectively. The composition of by-product extractant is estimated to be 70.7% fatty acid and 29.3% fatty acid isobutyl ester on a dry basis.

**Table 3**

| **Component** | **Mass %** |
|---|---|
| Water | 57.386% |
| Cells | 0.502% |
| Fatty acids | 6.737% |
| Isobutyl esters of fatty acids | 30.817% |
| Triglyceride | 0.035% |
| Suspended solids | 0.416% |
| Dissolved solids | 4.107% |

**Table 4**

| | **Hydrolyzer feed** | **Thin stillage** | **Solids** |
|---|---|---|---|
| Organics | 99.175% | 0.75% | 0.08% |
| Water and dissolved solids | 1% | 96% | 3% |
| Suspended solids and cells | 1% | 2% | 97% |

**Table 5**

| Stream | **C. protein** | **triglyceride** | **FFA** | **FABE** |
|---|---|---|---|---|
| Whole stillage wet cake | 40% | trace | 0.5% | 2.2% |
| Oil at evaporator | 0% | 0.08% | 16.1% | 73.8% |
| CDS | 22% | trace% | 0.37% | 1.71% |

### EXAMPLE 9

Example 9 provides an exemplary method and system showing combinations of process feedstreams that follow a process shown and as described herein.

Liquefied corn mash was prepared as described, for example, in Example 1, and corn oil and solids were removed as described, for example, in Examples 2, 3, and/or 5, forming corn oil and wet cake. Following fermentation as described, for example, in Example 4, solids were removed, forming wet cake as described, for example in Example 5. Syrup and lipid process feedstreams were produced as described herein. A process feedstream of fatty acids from hydrolyzing corn oil (COFA, process feedstream of fatty acids from hydrolyzing corn oil) was produced as described herein. The percentage by dry weight of triglycerides (TG), fatty acids (FA) and isobutyl esters of COFA (FABE) was determined for the process feedstreams as described, for example, in Examples 6 and 7. Crude fat, crude protein, lysine, neutral detergent fiber (NDF), and acid detergent fiber (ADF) percentages by dry weight, and dry material (DM) rate relative to wet cake were also determined for the process feedstreams using standard methods. In addition, these sample values were determined for three combinations of process feed streams: (1) wet cake, WS wet cake, syrup, and COFA (DCP1); (2) wet cake, WS wet cake, and syrup (DCP2); and (3) corn oil (65%), wet cake, WS wet cake, and syrup. Results of these analyses are shown in Table 6.

**Table 6: Process feedstream contents and combinations (percentage dry basis)**

| **Stream** | **Lipids** | | | **Total lipid** | | | | | |
|---|---|---|---|---|---|---|---|---|---|
| | **TG** | **FA** | **FABE** | **Crude fat** | **Crude protein** | **lysine** | **NDF** | **ADF** | **DM rate relative to wet cake** |
| Corn oil | 89 | 6 | 0 | 95 | 0 | 0 | --- | --- | |
| wet cake | 1.3 | 0.1 | 0 | 1.4 | 33.7 | 1.2 | 46 | 13 | 100 |
| WS wet cake | 0 | 0.47 | 2.16 | 2.6 | 38.7 | 1.8 | 19 | 5 | 29 |
| Lipid | 0 | 16 | 74 | 90 | 0 | 0 | --- | --- | 5 |
| Syrup | 0 | 0.4 | 1.7 | 2 | 21.6 | 0.8 | 4 | 3 | 108 |
| COFA | 0 | 71 | 29 | 100 | 0 | 0 | --- | --- | 24 |
| DCP1 | 0.5 | 6.7 | 3.6 | 10.8 | 26.2 | 1.0 | 21 | 7 | |
| DCP2 | 0.5 | 0.3 | 1.0 | 1.9 | 28.8 | 1.1 | 23 | 7 | |
| DCP3 | 6.2 | 0.9 | 1.0 | 8.1 | 27.0 | 1.0 | 22 | 7 | |

These results show that the process feedstreams can be used to generate components for a particular animal feed or animal feed market (e.g., livestock, ruminant, cattle, dairy animal, swine, goat, sheep, poultry, equine, aquaculture, domestic pet feed market). Further, combinations of process feedstreams can be used to optimize lipid, fat, protein or lysine content for a particular animal feed or animal feed market.

### Example 10

### Conversion of COFA to FAEE and monoacyl glycerol

The following examples show that COFA can be esterified with ethanol (EtOH) or with glycerol at high yield under mild conditions using immobilized enzyme.

Novozyme 435 (*Candida antarctica* lipase B, immobilized on an acrylic resin) was purchased from Sigma Aldrich (St. Louis, Mo). Acetone, *t*-BuOH, ethanol, methanol, and glycerol were all purchased from Sigma Aldrich (St. Louis, Mo). For GC analysis, the gas chromatograph used was Hewlett Packard 5890 Series II GC chromatogram and methyl pentadecanoate was used as an internal standard.

### Conversion of COFA to FAEE

Corn oil fatty acid (COFA, 0.25 g) was dissolved in 2.0 mL EtOH forming a single phase. Twenty mg of *Candida antarctica* lipase B (CALB) immobilized on acrylic resin (Novozyme 435) was added (contains 1.7 mg of enzyme) and the suspension was incubated for 24 h on a rotary shaker (300 rpm) at 40°C in a 6 mL glass vial sealed with a septum cap. The reaction went practically to completion with 98% of the COFA converted to FAEE (fatty acid ethyl ester). The GC analysis after 24 h showed 98% conversion of COFA to fatty acid ethyl ester.

### Conversion of COFA to monoacylglycerides

Corn oil fatty acid (COFA, 0.25 g) plus 0.325 g of glycerol were dissolved in 2.0 mL acetone. There was a large upper phase in which most of the components were dissolved and a small residual glycerol-containing phase. Twenty mg of *Candida antarctica* lipase B (CALB) immobilized on acrylic resin (Novozyme 435) was added (contains 1.7 mg of enzyme) and the suspension was incubated for 24 h on a rotary shaker (300 rpm) at 40°C in a 6 mL glass vial sealed with a septum cap. GC of the upper phase indicated that 87% of the COFA had been converted to acyl glyceride (expected to be mostly mono-acylglyceride).

### Example 11

### Conversion of COFA to FAME

The following examples show that COFA can be esterified with methanol (MeOH), with EtOH, and with glycerol at high yield under mild conditions using immobilized lipase.

### Conversion of COFA to FAME without solvent

To a 6 mL vial was added 500 mg COFA (1.48 mmol), 132 µL of MeOH (3.26 mmol), and 10 mg Novozyme 435. The resulting mixture was placed in an incubator/shaker, and left at 40°C overnight. GC analysis of the reaction mixture revealed 95% conversion.

### Adding MeOH to the COFA→FAME reaction

To a 6 mL vial was added 500 mg COFA (1.48 mmol), 180, 240, 300, or 1320 µL of MeOH (4.44, 5.92, 7.41, and 14.82 mmol), and 10 mg Novozyme 435. The resulting mixture was placed in an incubator/shaker, and left at 40°C overnight. GC analysis of the reaction mixture revealed 96-97% conversion.

While various embodiments of the present invention have been described above, it should be understood that they have been presented by way of example only, and not limitation. It will be apparent to persons skilled in the relevant art that various changes in form and detail can be made therein. Thus, the breadth and scope of the present invention should not be limited by any of the above-described exemplary embodiments, but should be defined only in accordance with the following claims.

## Claims

1. A method of generating a distillers co-products for animal feed comprising providing a butanol production process, wherein at least three process feedstreams are combined to generate a distillers co-products for animal feed having a crude protein content of at least about 20% by weight of the distillers co-products and wherein the at least three process feedstreams include syrup; solids from a mash before fermentation; and solids from a whole stillage after fermentation.

2. The method of claim 1, wherein the butanol is 1-butanol, 2-butanol, tertiary butanol and/or isobutanol either individually or as mixtures thereof.

3. The method of claim 1, wherein the microorganism is a recombinant microorganism that is genetically modified to produce butanol.

4. The method of claim 1, wherein the crude fat content of the distillers co-products is less than about 10% based on the weight of the distillers co-products.

5. The method of claim 1, wherein the yield of butanol is greater than about 8% by volume.

## Patentansprüche

1. Verfahren zum Erzeugen von Brennereinebenprodukten für Tierfutter, umfassend das Bereitstellen eines Butanolherstellungsverfahrens, wobei mindestens drei Prozesszuflüsse vereinigt werden, um ein Brennereinebenprodukt für Tierfutter zu erzeugen, das einen Rohproteingehalt von mindestens etwa 20 Gew.-%, auf die Brennereinebenprodukte bezogen, aufweist und wobei die mindestens drei Prozesszuflüsse Sirup, Feststoffe aus einer Maische vor der Fermentierung und Feststoffe aus einer Ganzschlempe nach dem Fermentieren umfassen.

2. Verfahren nach Anspruch 1, wobei das Butanol 1-Butanol, 2-Butanol, tertiäres Butanol und/oder Isobutanol entweder einzeln oder als Mischungen davon ist.

3. Verfahren nach Anspruch 1, wobei der Mikroorganismus ein rekombinanter Mikroorganismus ist, der genetisch modifiziert worden ist, um Butanol herzustellen.

4. Verfahren nach Anspruch 1, wobei der Rohfettgehalt der Brennereinebenprodukte weniger als etwa 10 %, auf das Gewicht der Brennereinebenprodukte bezogen, beträgt.

5. Verfahren nach Anspruch 1, wobei die Ausbeute an Butanol mehr als 8 Volumen-% beträgt.

## Revendications

1. Méthode pour la génération de co-produits de distillateurs pour l'alimentation animale comprenant la fourniture d'un procédé de production de butanol, dans laquelle au moins trois courants d'alimentation de procédé sont combinés pour générer des co-produits de distillateurs pour l'alimentation animale possédant une teneur en protéines brutes d'au moins environ 20% en poids des co-produits de distillateurs et dans laquelle les au moins trois courants d'alimentation de procédé incluent un sirop; des solides provenant d'une pâtée avant fermentation; et des solides provenant d'un jus de distillerie entier après fermentation.

2. Méthode selon la revendication 1, dans laquelle le butanol est le 1-butanol, le 2-butanol, le butanol tertiaire et/ou l'isobutanol soit individuellement, soit sous forme de mélanges de ceux-ci.

3. Méthode selon la revendication 1, dans laquelle le microorganisme est un microorganisme recombinant qui est génétiquement modifié pour produire du butanol.

4. Méthode selon la revendication 1, dans laquelle la teneur en graisse brute des co-produits de distillateurs est inférieure à environ 10% sur la base du poids des co-produits de distillateurs.

5. Méthode selon la revendication 1, dans laquelle le rendement de butanol est supérieur à environ 8% en volume.
